# EUROPEAN PATENT SPECIFICATION

(11) **EP 2 536 852 B1**
(45) Date of publication and mention of the grant of the patent: **18.09.2019**
(21) Application number: 11745050.2
(22) Date of filing: 09.02.2011
(51) Int. Cl.: C12Q 1/6809, C12Q 1/6883, G16H 50/00

(54) **METHODS FOR DETECTING FETAL NUCLEIC ACIDS AND DIAGNOSING FETAL ABNORMALITIES**
VERFAHREN ZUR ERKENNUNG FÖTALER NUKLEINSÄUREN UND ZUR DIAGNOSE FÖTALER ANOMALIEN
MÉTHODES DE DÉTECTION D'ACIDES NUCLÉIQUES FOETAUX ET DE DIAGNOSTIC D'ANOMALIES FOETALES

(30) Priority: 19.02.2010 US 709057; 19.03.2010 US 727824
(43) Date of publication of application: 26.12.2012
(73) Proprietor: Sequenom, Inc., San Diego, CA 92121 (US)
(72) Inventor: LAPIDUS, Stanley, N., Bedford, NH 03110 (US); THOMPSON, John, F., Warwick, RI 02886 (US); LIPSON, Doron, Chestnut Hill, MA 02467 (US); MILOS, Patrice, M., Cranston, RI 02905 (US); EFCAVITCH, J., William, San Carlos, CA 94070 (US); LETOVSKY, Stanley, Milton, MA 02186 (US)
(74) Representative: Bohmann, Armin K.
(86) International application number: PCT/US2011/024132
(87) International publication number: WO 2011/102998

(56) References cited:
- WO-A1-2011/091063
- WO-A2-2010/033578
- US-A1- 2008 020 390
- US-A1- 2008 138 809
- US-A1- 2009 317 817
- CAN ALKAN ET AL: "Personalized copy number and segmental duplication maps using next-generation sequencing", NATURE GENETICS, vol. 41, no. 10, 30 October 2009 (2009-10-30), pages 1061-1067, XP055147400, ISSN: 1061-4036, DOI: 10.1038/ng.437 & Can Alkan ET AL: "Supplementary Information - Personalized copy number and segmental duplication maps using next-generation sequencing", Nature Genetics, vol. 41, no. 10, 30 August 2009 (2009-08-30), pages 1-68, XP055204506, ISSN: 1061-4036, DOI: 10.1038/ng.437
- T. CHU ET AL: "Statistical model for whole genome sequencing and its application to minimally invasive diagnosis of fetal genetic disease", BIOINFORMATICS, vol. 25, no. 10, 23 March 2009 (2009-03-23), pages 1244-1250, XP055018601, ISSN: 1367-4803, DOI: 10.1093/bioinformatics/btp156
- FAN H C ET AL: "Noninvasive diagnosis of fetal aneuploidy by shotgun sequencing DNA from maternal blood", PROCEEDINGS OF THE NATIONAL ACADEMY OF SCIENCES, NATIONAL ACADEMY OF SCIENCES, US, vol. 105, no. 42, 21 October 2008 (2008-10-21), pages 16266-16271, XP002613056, ISSN: 0027-8424, DOI: 10.1073/PNAS.0808319105 [retrieved on 2008-10-06]
- J. C. DOHM ET AL: "Substantial biases in ultra-short read data sets from high-throughput DNA sequencing", NUCLEIC ACIDS RESEARCH, vol. 36, no. 16, 1 August 2008 (2008-08-01), pages e105-e105, XP055204412, ISSN: 0305-1048, DOI: 10.1093/nar/gkn425
- CHIU ROSSA W K ET AL: "Noninvasive prenatal diagnosis of fetal chromosomal aneuploidy by massively parallel genomic sequencing of DNA in maternal plasma", PROCEEDINGS OF THE NATIONAL ACADEMY OF SCIENCES, NATIONAL ACADEMY OF SCIENCES, US, vol. 105, no. 51, 23 December 2008 (2008-12-23), pages 20458-20463, XP002620454, ISSN: 0027-8424, DOI: 10.1073/PNAS.0810641105 [retrieved on 2008-12-10] & R. W. K. Chiu ET AL: "Supplementary Information for XP002620454 (Title: Noninvasive prenatal diagnosis of fetal chromosomal aneuploidy by massively parallel genomic sequencing of DNA in maternal plasma)", Proceedings of the National Academy of Sciences, vol. 105, no. 51, 10 December 2008 (2008-12-10), pages 20458-20463, XP055024153, US ISSN: 0027-8424, DOI: 10.1073/pnas.0810641105
- T. D. HARRIS ET AL: "Single-Molecule DNA Sequencing of a Viral Genome", SCIENCE, vol. 320, no. 5872, 4 April 2008 (2008-04-04), pages 106-109, XP055072779, ISSN: 0036-8075, DOI: 10.1126/science.1150427
- H. CHRISTINA FAN ET AL: "Sensitivity of Noninvasive Prenatal Detection of Fetal Aneuploidy from Maternal Plasma Using Shotgun Sequencing Is Limited Only by Counting Statistics", PLOS ONE, vol. 5, no. 5, 3 May 2010 (2010-05-03), page e10439, XP055026436, ISSN: 1932-6203, DOI: 10.1371/journal.pone.0010439
- FAN, H.C. ET AL.: 'Noninvasive diagnosis of fatal aneuploidy by shotgun sequencing DNA from matemal blood.' PROC. NAT. ACAD. SCI. vol. 105, no. 42, 21 October 2008, pages 16266 - 16271, XP002613056
- PUSHKAREV, D. ET AL.: 'Single-molecule sequencing of an individual human genome' NATURE BIOTECHNOLOGY vol. 27, no. 9, 10 August 2009, pages 847 - 852, XP055136148

## Description

### Field of the Invention

The invention is related to a method for analyzing nucleic acids in a sample.

### Background

Fetal aneuploidy (e.g., Down syndrome, Edward syndrome, and Patau syndrome) and other chromosomal aberrations affect 9 of 1,000 live births (Cunningham et al. in Williams Obstetrics, McGraw-Hill, New York, p. 942, 2002). Chromosomal abnormalities are generally diagnosed by karyotyping of fetal cells obtained by invasive procedures such as chorionic villus sampling or amniocentesis. Those procedures are associated with potentially significant risks to both the fetus and the mother Can Alkan et al. ( Nature Genetics, vol. 41(10), 30 October 2009, pages 1061-1067) report on personalized copy number and segmental duplication maps using next-generation sequencing. Noninvasive screening using maternal serum markers or ultrasound are available but have limited reliability (Fan et al., PNAS, 105(42):16266-16271, 2008).

Since the discovery of intact fetal cells in maternal blood, there has been intense interest in trying to use those cells as a diagnostic window into fetal genetics (Fan et al., PNAS, 105(42):16266-16271, 2008). The discovery that certain amounts (between about 3% and about 6%) of cell-free fetal nucleic acids exist in maternal circulation has led to the development of noninvasive PCR based prenatal genetic tests for a variety of traits. A problem with those tests is that PCR based assays trade off sensitivity for specificity, making it difficult to identify particular mutations. Further, due to the stochastic nature of PCR, a population of molecules that is present in a small amount in the sample often is overlooked, such as fetal nucleic acid in a sample from a maternal tissue or body fluid. In fact, if rare nucleic acid is not amplified in the first few rounds of amplification, it becomes increasingly unlikely that the rare event will ever be detected.

Additionally, there is also the potential that fetal nucleic acid in a maternal sample is degraded and not amendable to PCR amplification due to the small size of the nucleic acid.

There is a need for methods that can noninvasively detect fetal nucleic acids and diagnose fetal abnormalities.

### Summary

The problem underlying the present invention is solved by the subject matter of the attached independent claim. Preferred embodiments may be taken from the attached dependent claims.

More specifically, the problem underlying the present invention is solved by a method for analyzing nucleic acids in a sample, the method comprising:
sequencing a sample to obtain nucleic acid sequence information;
determining an amount of GC bias in the sequence information, wherein the determining comprises:
   partitioning the genome into bins; and
   measuring a correlation between a number of counts in each bin and its GC content, wherein a statistically significant negative or positive correlation indicates existence of GC bias;
correcting the sequence information to account for the GC bias by selecting a subset of bins within a given range of GC content such that average GC content per chromosome is equalized; and
analyzing the corrected sequence information by performing chromosomal counting using the counts of the selected subset of bins.

In an embodiment, the method further comprises
comparing corrected sequence information to a reference sequence;
identifying fetal nucleic acid in the sample;
determining whether the fetus has an abnormality based on the comparison to the reference sequence.

In an embodiment, the bins in the selected subset of bins have a range of GC content of about 0.42 to 0.48.

In an embodiment, the selected subset of bins represents about 25 % of a reference genome.

In an embodiment, the size of a bin is about 1000 kilobase pairs (kb).

In an embodiment, the sample is a tissue or body fluid.

In an embodiment, the sample is suspected to contain fetal nucleic acid.

In an embodiment, the body fluid is maternal blood, blood plasma, or serum.

In an embodiment, sequencing is single molecule sequencing and/or sequencing by nanopore detection.

In an embodiment, the reference sequence is selected from a maternal reference sequence, a fetal reference sequence, or a consensus human genomic sequence.

In an embodiment, said maternal reference sequence is selected from a sequence obtained from a buccal sample, a saliva sample, a urine sample, a breast nipple aspirate sample, a sputum sample, a tear sample, and an amniotic fluid sample.

In an embodiment, the fetal nucleic acid is cell free circulating fetal nucleic acid.

In an embodiment, prior to the sequencing step, the method further comprises enriching for fetal nucleic acid in the sample.

In an embodiment, the identifying step comprises a technique selected from sparse allele calling, targeted gene sequencing, identification of Y chromosomal material, enumeration, copy number analysis, and inversion analysis.

Methods of the invention take advantage of sequencing technologies, particularly single molecule sequencing-by-synthesis technologies, to detect fetal nucleic acid in maternal tissues or body fluids. Methods of the invention are highly sensitive and allow for the detection of the small population of fetal nucleic acids in a maternal sample, generally without the need for amplification of the nucleic acid in the sample.

Methods of the invention involve sequencing nucleic acid obtained from a maternal sample and distinguishing between maternal and fetal nucleic acid. Distinguishing between maternal and fetal nucleic acid identifies fetal nucleic acid, thus allowing the determination of abnormalities based upon sequence variation. Such abnormalities may be determined as single nucleotide polymorphisms, variant motifs, inversions, deletions, additions, or any other nucleic acid rearrangement or abnormality. In an embodiment of the invention as defined in the claims, the presence of fetal nucleic acid in a maternal sample may be determined by identifying nucleic acid that is unique to the fetus. For example, one can look for differences between obtained sequence and maternal reference sequence; or can involve the identification of Y chromosomal material in the sample. The maternal sample may be a tissue or body fluid. In particular embodiments, the body fluid is maternal blood, maternal blood plasma, or maternal serum.

In an embodiment of the invention as defined in the claims, the presence of fetal nucleic acid in a maternal sample may be confirmed by, for example, looking for unique sequences or variants.

The sequencing reaction may be any sequencing reaction. In particular embodiments, the sequencing reaction is a single molecule sequencing reaction. Single-molecule sequencing is shown for example in Lapidus et al. (U.S. patent number 7,169,560), Lapidus et al. (U.S. patent application number 2009/0191565), Quake et al. (U.S. patent number 6,818,395), Harris (U.S. patent number 7,282,337), Quake et al. (U.S. patent application number 2002/0164629), and Braslavsky, et al., PNAS (USA), 100: 3960-3964 (2003).

Briefly, in some implementations, a single-stranded nucleic acid (e.g., DNA or cDNA) is hybridized to oligonucleotides attached to a surface of a flow cell. The oligonucleotides may be covalently attached to the surface or various attachments other than covalent linking as known to those of ordinary skill in the art may be employed. Moreover, the attachment may be indirect, e.g., via the polymerases of the invention directly or indirectly attached to the surface. The surface may be planar or otherwise, and/or may be porous or non-porous, or any other type of surface known to those of ordinary skill to be suitable for attachment. The nucleic acid is then sequenced by imaging or otherwise detecting the polymerase-mediated addition of fluorescently-labeled nucleotides incorporated into the growing strand surface oligonucleotide, at single molecule resolution. In certain embodiments, the nucleotides used in the sequencing reaction are not chain terminating nucleotides.

Because the Y chromosome will only be present if the fetal nucleic acid is from a male, methods of the invention may further include performing a quantitative assay on the obtained sequences to detect presence of fetal nucleic acid if the Y chromosome is not detected in the sample. Such quantitative assays include copy number analysis, sparse allele calling, targeted resequencing, and breakpoint analysis.

The ability to detect fetal nucleic acid in a maternal sample allows for development of a noninvasive diagnostic assay to assess whether a fetus has an abnormality. Thus, another aspect of the invention provides noninvasive methods for determining whether a fetus has an abnormality. Methods of the invention may involve obtaining a sample including both maternal and fetal nucleic acids, performing a sequencing reaction on the sample to obtain sequence information on nucleic acids in the sample, comparing the obtained sequence information to sequence information from a reference genome, thereby determining whether the fetus has an abnormality, detecting presence of at least a portion of a Y chromosome in the sample, and distinguishing false negatives from true negatives if the Y chromosome is not detected in the sample.

An important aspect of a diagnostic assay is the ability of the assay to distinguish between false negatives (no detection of fetal nucleic acid when in fact it is present) and true negatives (detection of nucleic acid from a healthy fetus). In an embodiment of the invention as defined in the claims, this capability is provided. If the Y chromosome is detected in the maternal sample, it is assured that the assay is functioning properly, because the Y chromosome is associated only with males and will be present in a maternal sample only if male fetal nucleic acid is present in the sample. In an embodiment of the invention as defined in the claims, further quantitative or qualitative analysis to distinguish between false negatives and true negative is provided, regardless of the ability to detect the Y chromosome, particularly for samples including normal nucleic acids from a female fetus. Such additional quantitative analysis may include copy number analysis, sparse allele calling, targeted resequencing, and breakpoint analysis.

In an embodiment of the invention as defined in the claims, it is determined whether a fetus has an abnormality, including obtaining a maternal sample comprising both maternal and fetal nucleic acids; attaching unique tags to nucleic acids in the sample, in which each tag is associated with a different chromosome; performing a sequencing reaction on the tagged nucleic acids to obtain tagged sequences; and determining whether the fetus has an abnormality by quantifying the tagged sequences. In certain embodiments, the tags include unique nucleic acid sequences.

### Brief Description of the Drawings

Figure 1 is a histogram showing difference between one individual ("self') and two family members ("family") representing a comparison of a set of known single nucleotide variants between the three samples.
Figure 2 is a table showing HapMap DNA sequence reads derived from single molecule sequencing and aligned uniquely to a reference human genome. Each column represents data from a single HELISCOPE sequencer (Single molecule sequencing apparatus, Helicos BioSciences Corporation) channel.
Figure 3 is a table showing normalized chromosomal reads per sample. The individual chromosomal counts were divided by total autosomal counts.
Figure 4 is a table showing normalized counts per chromosome. The average fraction of reads aligned to each chromosome across all samples.
Figure 5 is a graphic representation of quantitative chromosomal counts.
Figure 6 is a graph showing a sample in which chromosomal counts are skewed by GC bias.
Figure 7 is a graph showing genomic bins plotted as a function of GC content in the bin. In Figure 7, the upper sample shows positive correlation with GC content, and the lower sample shows negative correlation with GC content.
Figure 8 panel A is a graph showing selection of certain genomic bins with a given GC content for analysis. Figure 8 panel B shows the sequence information prior to correction for GC bias. Figure 8 panel C shows the sequence information after correction for GC bias.
Figure 9 panels A and B show sequence information prior to correction for GC bias. Figure 9 panels C and D show sequence information after correction for GC bias.
Figure 10 shows results of analysis of the sequence information.

### Detailed Description

Methods of the invention use sequencing reactions in order to detect presence of fetal nucleic acid in a maternal sample. Methods of the invention also use sequencing reactions to analyze maternal blood for a genetic condition, in which mixed fetal and maternal nucleic acid in the maternal blood is analyzed to distinguish a fetal mutation or genetic abnormality from a background of the maternal nucleic acid.

Fetal nucleic acid includes both fetal DNA and fetal RNA. As described in Ng et al., mRNA of placental origin is readily detectable in maternal plasma, Proc. Nat. Acad. Sci. 100(8): 4748-4753 (2003).

### Samples

Methods of the invention involve obtaining a sample, e.g., a tissue or body fluid, that is suspected to include both maternal and fetal nucleic acids. Such samples may include saliva, urine, tear, vaginal secretion, amniotic fluid, breast fluid, breast milk, sweat, or tissue. In certain embodiments, this sample is drawn maternal blood, and circulating DNA is found in the blood plasma, rather than in cells. A preferred sample is maternal peripheral venous blood.

In certain embodiments, approximately 10-20 mL of blood is drawn. That amount of blood allows one to obtain at least about 10,000 genome equivalents of total nucleic acid (sample size based on an estimate of fetal nucleic acid being present at roughly 25 genome equivalents/mL of maternal plasma in early pregnancy, and a fetal nucleic acid concentration of about 3.4% of total plasma nucleic acid). However, less blood may be drawn for a genetic screen where less statistical significance is required, or the nucleic acid sample is enriched for fetal nucleic acid.

Because the amount of fetal nucleic acid in a maternal sample generally increases as a pregnancy progresses, less sample may be required as the pregnancy progresses in order to obtain the same or similar amount of fetal nucleic acid from a sample.

### Enrichment

In certain embodiments, the sample (e.g., blood, plasma, or serum) may optionally be enriched for fetal nucleic acid by known methods, such as size fractionation to select for DNA fragments less than about 300 bp. Alternatively, maternal DNA, which tends to be larger than about 500 bp, may be excluded.

In certain embodiments, the maternal blood may be processed to enrich the fetal DNA concentration in the total DNA, as described in Li et al., J. Amer. Med. Assoc. 293:843-849, 2005), the contents of which are incorporated by reference herein in their entirety. Briefly, circulatory DNA is extracted from 5 mL to 10 mL maternal plasma using commercial column technology (Roche High Pure Template DNA Purification Kit; Roche, Basel, Switzerland) in combination with a vacuum pump. After extraction, the DNA is separated by agarose gel (1%) electrophoresis (Invitrogen, Basel, Switzerland), and the gel fraction containing circulatory DNA with a size of approximately 300 bp is carefully excised. The DNA is extracted from this gel slice by using an extraction kit (QIAEX II Gel Extraction Kit; Qiagen, Basel, Switzerland) and eluted into a final volume of 40 µL sterile 10-mM trishydrochloric acid, pH 8.0 (Roche).

DNA may be concentrated by known methods, including centrifugation and various enzyme inhibitors. The DNA is bound to a selective membrane (e.g., silica) to separate it from contaminants. The DNA is preferably enriched for fragments circulating in the plasma, which are less than 1000 base pairs in length, generally less than 300 bp. This size selection is done on a DNA size separation medium, such as an electrophoretic gel or chromatography material. Such a material is described in Huber et al. (Nucleic Acids Res. 21(5):1061-1066, 1993), gel filtration chromatography, TSK gel, as described in Kato et al., (J. Biochem, 95(1):83-86, 1984).

In addition, enrichment may be accomplished by suppression of certain alleles through the use of peptide nucleic acids (PNAs), which bind to their complementary target sequences, but do not amplify.

Plasma RNA extraction is described in Enders et al. (Clinical Chemistry 49:727-731, 2003), the contents of which are incorporated by reference herein in their entirety. As described there, plasma harvested after centrifugation steps is mixed with Trizol LS reagent (Invitrogen) and chloroform. The mixture is centrifuged, and the aqueous layer transferred to new tubes. Ethanol is added to the aqueous layer. The mixture is then applied to an RNeasy mini column (Qiagen) and processed according to the manufacturer's recommendations.

Another enrichment step may be to treat the blood sample with formaldehyde, as described in Dhallan et al. (J. Am. Med. Soc. 291(9): 1114-1119, March 2004; and U.S. patent application number 20040137470). Dhallan et al. (U.S. patent application number 20040137470) describes an enrichment procedure for fetal DNA, in which blood is collected into 9 ml EDTA Vacuette tubes (catalog number NC9897284) and 0.225 ml of 10% neutral buffered solution containing formaldehyde (4% w/v), is added to each tube, and each tube gently is inverted. The tubes are stored at 4°C. until ready for processing.

Agents that impede cell lysis or stabilize cell membranes can be added to the tubes including but not limited to formaldehyde, and derivatives of formaldehyde, formalin, glutaraldehyde, and derivatives of glutaraldehyde, crosslinkers, primary amine reactive crosslinkers, sulfhydryl reactive crosslinkers, sulfhydryl addition or disulfide reduction, carbohydrate reactive crosslinkers, carboxyl reactive crosslinkers, photoreactive crosslinkers, cleavable crosslinkers, etc. Any concentration of agent that stabilizes cell membranes or impedes cell lysis can be added. In certain embodiments, the agent that stabilizes cell membranes or impedes cell lysis is added at a concentration that does not impede or hinder subsequent reactions.

Flow cytometry techniques can also be used to enrich fetal cells (Herzenberg et al., PNAS 76:1453-1455, 1979; Bianchi et al., PNAS 87:3279-3283, 1990; Bruch et al., Prenatal Diagnosis 11:787-798, 1991). Saunders et al. (U.S. patent number 5,432,054) also describes a technique for separation of fetal nucleated red blood cells, using a tube having a wide top and a narrow, capillary bottom made of polyethylene. Centrifugation using a variable speed program results in a stacking of red blood cells in the capillary based on the density of the molecules. The density fraction containing low-density red blood cells, including fetal red blood cells, is recovered and then differentially hemolyzed to preferentially destroy maternal red blood cells. A density gradient in a hypertonic medium is used to separate red blood cells, now enriched in the fetal red blood cells from lymphocytes and ruptured maternal cells. The use of a hypertonic solution shrinks the red blood cells, which increases their density, and facilitates purification from the more dense lymphocytes. After the fetal cells have been isolated, fetal DNA can be purified using standard techniques in the art.

Further, an agent that stabilizes cell membranes may be added to the maternal blood to reduce maternal cell lysis including but not limited to aldehydes, urea formaldehyde, phenol formaldehyde, DMAE (dimethylaminoethanol), cholesterol, cholesterol derivatives, high concentrations of magnesium, vitamin E, and vitamin E derivatives, calcium, calcium gluconate, taurine, niacin, hydroxylamine derivatives, bimoclomol, sucrose, astaxanthin, glucose, amitriptyline, isomer A hopane tetral phenylacetate, isomer B hopane tetral phenylacetate, citicoline, inositol, vitamin B, vitamin B complex, cholesterol hemisuccinate, sorbitol, calcium, coenzyme Q, ubiquinone, vitamin K, vitamin K complex, menaquinone, zonegran, zinc, ginkgo biloba extract, diphenylhydantoin, perftoran, polyvinylpyrrolidone, phosphatidylserine, tegretol, PABA, disodium cromglycate, nedocromil sodium, phenyloin, zinc citrate, mexitil, dilantin, sodium hyaluronate, or polaxamer 188.

An example of a protocol for using this agent is as follows: The blood is stored at 4°C. until processing. The tubes are spun at 1000 rpm for ten minutes in a centrifuge with braking power set at zero. The tubes are spun a second time at 1000 rpm for ten minutes. The supernatant (the plasma) of each sample is transferred to a new tube and spun at 3000 rpm for ten minutes with the brake set at zero. The supernatant is transferred to a new tube and stored at -80°C. Approximately two milliliters of the "buffy coat," which contains maternal cells, is placed into a separate tube and stored at -80°C.

Genomic DNA may be isolated from the plasma using the Qiagen Midi Kit for purification of DNA from blood cells, following the manufacturer's instructions (QIAmp DNA Blood Midi Kit, Catalog number 51183). DNA is eluted in 100 µl of distilled water. The Qiagen Midi Kit also is used to isolate DNA from the maternal cells contained in the "buffy coat."

### Extraction

Nucleic acid is extracted from the sample according to methods known in the art. See for example, Maniatis, et al., Molecular Cloning: A Laboratory Manual, Cold Spring Harbor, N.Y., pp. 280-281, 1982.

### Determining presence of male fetal nucleic acid in a maternal sample

The nucleic acid from the sample is then analyzed using a sequencing reaction in order to detect presence of at least a portion of a Y chromosome in the sample. For example, Bianchi et al. (PNAS USA, 87:3279-3283, 1990) reports a 222 bp sequence that is present only on the short arm of the Y chromosome. Lo et al. (Lancet, 350:485-487, 1997), Lo, et al., (Am J Hum Genet, 62(4):768, 1998), and Smid et al. (Clin Chem, 45:1570-1572, 1999) each reports different Y-chromosomal sequences derived from male fetuses. The contents of each of these articles is incorporated by reference herein in their entirety. If the Y chromosome is detected in the maternal sample, methods of the invention assure that the sample includes fetal nucleic acid, because the Y chromosome is associated only with males and will be present in a maternal sample only if male fetal nucleic acid is present in the sample.

In certain embodiments, the sequencing method is a single molecule sequencing by synthesis method. Single molecule sequencing is shown for example in Lapidus et al. (U.S. patent number 7,169,560), Lapidus et al. (U.S. patent application number 2009/0191565), Quake et al. (U.S. patent number 6,818,395), Harris (U.S. patent number 7,282,337), Quake et al. (U.S. patent application number 2002/0164629), and Braslavsky, et al., PNAS (USA), 100: 3960-3964 (2003).

Briefly, a single-stranded nucleic acid (e.g., DNA or cDNA) is hybridized to oligonucleotides attached to a surface of a flow cell. The oligonucleotides may be covalently attached to the surface or various attachments other than covalent linking as known to those of ordinary skill in the art may be employed. Moreover, the attachment may be indirect, e.g., via a polymerase directly or indirectly attached to the surface. The surface may be planar or otherwise, and/or may be porous or non-porous, or any other type of surface known to those of ordinary skill to be suitable for attachment. The nucleic acid is then sequenced by imaging the polymerase-mediated addition of fluorescently-labeled nucleotides incorporated into the growing strand surface oligonucleotide, at single molecule resolution. In certain embodiments, the nucleotides used in the sequencing reaction are not chain terminating nucleotides. The following sections discuss general considerations for nucleic acid sequencing, for example, polymerases useful in sequencing-by-synthesis, choice of surfaces, reaction conditions, signal detection and analysis.

### Nucleotides

Nucleotides useful in the invention include any nucleotide or nucleotide analog, whether naturally-occurring or synthetic. For example, preferred nucleotides include phosphate esters of deoxyadenosine, deoxycytidine, deoxyguanosine, deoxythymidine, adenosine, cytidine, guanosine, and uridine. Other nucleotides useful in the invention comprise an adenine, cytosine, guanine, thymine base, a xanthine or hypoxanthine; 5-bromouracil, 2-aminopurine, deoxyinosine, or methylated cytosine, such as 5-methylcytosine, and N4-methoxydeoxycytosine. Also included are bases of polynucleotide mimetics, such as methylated nucleic acids, e.g., 2'-O-methRNA, peptide nucleic acids, modified peptide nucleic acids, locked nucleic acids and any other structural moiety that can act substantially like a nucleotide or base, for example, by exhibiting base-complementarity with one or more bases that occur in DNA or RNA and/or being capable of base-complementary incorporation, and includes chain-terminating analogs. A nucleotide corresponds to a specific nucleotide species if they share base-complementarity with respect to at least one base.

Nucleotides for nucleic acid sequencing according to the invention preferably include a detectable label that is directly or indirectly detectable. Preferred labels include optically-detectable labels, such as fluorescent labels. Examples of fluorescent labels include, but are not limited to, Atto dyes, 4-acetamido-4'-isothiocyanatostilbene-2,2'disulfonic acid; acridine and derivatives: acridine, acridine isothiocyanate; 5-(2'-aminoethyl)aminonaphthalene-1-sulfonic acid (EDANS); 4-amino-N-[3-vinylsulfonyl)phenyl]naphthalimide-3,5 disulfonate; N-(4-anilino-1-naphthyl)maleimide; anthranilamide; BODIPY; Brilliant Yellow; coumarin and derivatives; coumarin, 7-amino-4-methylcoumarin (AMC, Coumarin 120), 7-amino-4-trifluoromethylcouluarin (Coumaran 151); cyanine dyes; cyanosine; 4',6-diaminidino-2-phenylindole (DAPI); 5'5"-dibromopyrogallol-sulfonaphthalein (Bromopyrogallol Red); 7-diethylamino-3-(4'-isothiocyanatophenyl)-4-methylcoumarin; diethylenetriamine pentaacetate; 4,4'-diisothiocyanatodihydro-stilbene-2,2'-disulfonic acid; 4,4'-diisothiocyanatostilbene-2,2'-disulfonic acid; 5-[dimethylamino]naphthalene-1-sulfonyl chloride (DNS, dansylchloride); 4-dimethylaminophenylazophenyl-4'-isothiocyanate (DABITC); eosin and derivatives; eosin, eosin isothiocyanate, erythrosin and derivatives; erythrosin B, erythrosin, isothiocyanate; ethidium; fluorescein and derivatives; 5-carboxyfluorescein (FAM), 5-(4,6-dichlorotriazin-2-yl)aminofluorescein (DTAF), 2',7'-dimethoxy-4'5'-dichloro-6-carboxyfluorescein, fluorescein, fluorescein isothiocyanate, QFITC, (XRITC); fluorescamine; IR144; IR1446; Malachite Green isothiocyanate; 4-methylumbelliferoneortho cresolphthalein; nitrotyrosine; pararosaniline; Phenol Red; B-phycoerythrin; o-phthaldialdehyde; pyrene and derivatives: pyrene, pyrene butyrate, succinimidyl 1-pyrene; butyrate quantum dots; Reactive Red 4 (Cibacron.TM. Brilliant Red 3B-A) rhodamine and derivatives: 6-carboxy-X-rhodamine (ROX), 6-carboxyrhodamine (R6G), lissamine rhodamine B sulfonyl chloride rhodamine (Rhod), rhodamine B, rhodamine 123, rhodamine X isothiocyanate, sulforhodamine B, sulforhodamine 101, sulfonyl chloride derivative of sulforhodamine 101 (Texas Red); N,N,N',N'tetramethyl-6-carboxyrhodamine (TAMRA); tetramethyl rhodamine; tetramethyl rhodamine isothiocyanate (TRITC); riboflavin; rosolic acid; terbium chelate derivatives; Cy3; Cy5; Cy5.5; Cy7; IRD 700; IRD 800; La Jolta Blue; phthalo cyanine; and naphthalo cyanine. Preferred fluorescent labels are cyanine-3 and cyanine-5. Labels other than fluorescent labels are contemplated by the invention, including other optically-detectable labels.

### Polymerases

Nucleic acid polymerases generally useful in the invention include DNA polymerases, RNA polymerases, reverse transcriptases, and mutant or altered forms of any of the foregoing. DNA polymerases and their properties are described in detail in, among other places, DNA Replication 2nd edition, Kornberg and Baker, W. H. Freeman, New York, N.Y. (1991). Known conventional DNA polymerases useful in the invention include, but are not limited to, Pyrococcus furiosus (Pfu) DNA polymerase (Lundberg et al., 1991, Gene, 108: 1, Stratagene), Pyrococcus woesei (Pwo) DNA polymerase (Hinnisdaels et al., 1996, Biotechniques, 20:186-8, Boehringer Mannheim), Thermus thermophilus (Tth) DNA polymerase (Myers and Gelfand 1991, Biochemistry 30:7661), Bacillus stearothermophilus DNA polymerase (Stenesh and McGowan, 1977, Biochim Biophys Acta 475:32), Thermococcus litoralis (Tli) DNA polymerase (also referred to as Vent.TM. DNA polymerase, Cariello et al., 1991, Polynucleotides Res, 19: 4193, New England Biolabs), 9.degree.Nm.TM. DNA polymerase (New England Biolabs), Stoffel fragment, ThermoSequenase® (Amersham Pharmacia Biotech UK), Therminator.TM. (New England Biolabs), Thermotoga maritima (Tma) DNA polymerase (Diaz and Sabino, 1998 Braz J. Med. Res, 31:1239), Thermus aquaticus (Taq) DNA polymerase (Chien et al., 1976, J. Bacteoriol, 127: 1550), DNA polymerase, Pyrococcus kodakaraensis KOD DNA polymerase (Takagi et al., 1997, Appl. Environ. Microbiol. 63:4504), JDF-3 DNA polymerase (from thermococcus sp. JDF-3, Patent application WO 0132887), Pyrococcus GB-D (PGB-D) DNA polymerase (also referred as Deep Vent.TM. DNA polymerase, Juncosa-Ginesta et al., 1994, Biotechniques, 16:820, New England Biolabs), UlTma DNA polymerase (from thermophile Thermotoga maritima; Diaz and Sabino, 1998 Braz J. Med. Res, 31:1239; PE Applied Biosystems), Tgo DNA polymerase (from thermococcus gorgonarius, Roche Molecular Biochemicals), E. coli DNA polymerase I (Lecomte and Doubleday, 1983, Polynucleotides Res. 11:7505), T7 DNA polymerase (Nordstrom et al., 1981, J. Biol. Chem. 256:3112), and archaeal DP1I/DP2 DNA polymerase II (Cann et al, 1998, Proc. Natl. Acad. Sci. USA 95:14250).

Both mesophilic polymerases and thermophilic polymerases are contemplated. Thermophilic DNA polymerases include, but are not limited to, ThermoSequenase®, 9.degree.Nm.TM., Therminator.TM., Taq, Tne, Tma, Pfu, Tfl, Tth, Tli, Stoffel fragment, Vent.TM. and Deep Vent.TM. DNA polymerase, KOD DNA polymerase, Tgo, JDF-3, and mutants, variants and derivatives thereof. A highly-preferred form of any polymerase is a 3' exonuclease-deficient mutant.

Reverse transcriptases useful in the invention include, but are not limited to, reverse transcriptases from HIV, HTLV-1, HTLV-II, FeLV, FIV, SIV, AMV, MMTV, MoMuLV and other retroviruses (see Levin, Cell 88:5-8 (1997); Verma, Biochim Biophys Acta. 473:1-38 (1977); Wu et al., CRC Crit. Rev Biochem. 3:289-347 (1975)).

### Attachment

In a preferred embodiment, nucleic acid template molecules are attached to a substrate (also referred to herein as a surface) and subjected to analysis by single molecule sequencing as described herein. Nucleic acid template molecules are attached to the surface such that the template/primer duplexes are individually optically resolvable. Substrates for use in the invention can be two- or three-dimensional and can comprise a planar surface (e.g., a glass slide) or can be shaped. A substrate can include glass (e.g., controlled pore glass (CPG)), quartz, plastic (such as polystyrene (low cross-linked and high cross-linked polystyrene), polycarbonate, polypropylene and poly(methymethacrylate)), acrylic copolymer, polyamide, silicon, metal (e.g., alkanethiolate-derivatized gold), cellulose, nylon, latex, dextran, gel matrix (e.g., silica gel), polyacrolein, or composites.

Suitable three-dimensional substrates include, for example, spheres, microparticles, beads, membranes, slides, plates, micromachined chips, tubes (e.g., capillary tubes), microwells, microfluidic devices, channels, filters, or any other structure suitable for anchoring a nucleic acid. Substrates can include planar arrays or matrices capable of having regions that include populations of template nucleic acids or primers. Examples include nucleoside-derivatized CPG and polystyrene slides; derivatized magnetic slides; polystyrene grafted with polyethylene glycol, and the like.

Substrates are preferably coated to allow optimum optical processing and nucleic acid attachment. Substrates for use in the invention can also be treated to reduce background. Exemplary coatings include epoxides, and derivatized epoxides (e.g., with a binding molecule, such as an oligonucleotide or streptavidin).

Various methods can be used to anchor or immobilize the nucleic acid molecule to the surface of the substrate. The immobilization can be achieved through direct or indirect bonding to the surface. The bonding can be by covalent linkage. See, Joos et al., Analytical Biochemistry 247:96-101, 1997; Oroskar et al., Clin. Chem. 42:1547-1555, 1996; and Khandjian, Mol. Bio. Rep. 11:107-115, 1986. A preferred attachment is direct amine bonding of a terminal nucleotide of the template or the 5' end of the primer to an epoxide integrated on the surface. The bonding also can be through non-covalent linkage. For example, biotin-streptavidin (Taylor et al., J. Phys. D. Appl. Phys. 24:1443, 1991) and digoxigenin with anti-digoxigenin (Smith et al., Science 253:1122, 1992) are common tools for anchoring nucleic acids to surfaces and parallels. Alternatively, the attachment can be achieved by anchoring a hydrophobic chain into a lipid monolayer or bilayer. Other methods for known in the art for attaching nucleic acid molecules to substrates also can be used.

### Detection

Any detection method can be used that is suitable for the type of label employed. Thus, exemplary detection methods include radioactive detection, optical absorbance detection, e.g., UV-visible absorbance detection, optical emission detection, e.g., fluorescence or chemiluminescence. For example, extended primers can be detected on a substrate by scanning all or portions of each substrate simultaneously or serially, depending on the scanning method used. For fluorescence labeling, selected regions on a substrate may be serially scanned one-by-one or row-by-row using a fluorescence microscope apparatus, such as described in Fodor (U.S. Pat. No. 5,445,934) and Mathies et al. (U.S. Pat. No. 5,091,652). Devices capable of sensing fluorescence from a single molecule include scanning tunneling microscope (siM) and the atomic force microscope (AFM). Hybridization patterns may also be scanned using a CCD camera (e.g., Model TE/CCD512SF, Princeton Instruments, Trenton, N.J.) with suitable optics (Ploem, in Fluorescent and Luminescent Probes for Biological Activity Mason, T. G. Ed., Academic Press, Landon, pp. 1-11 (1993), such as described in Yershov et al., Proc. Natl. Acad. Sci. 93:4913 (1996), or may be imaged by TV monitoring. For radioactive signals, a phosphorimager device can be used (Johnston et al., Electrophoresis, 13:566, 1990; Drmanac et al., Electrophoresis, 13:566, 1992; 1993). Other commercial suppliers of imaging instruments include General Scanning Inc., (Watertown, Mass. on the World Wide Web at genscan.com), Genix Technologies (Waterloo, Ontario, Canada; on the World Wide Web at confocal.com), and Applied Precision Inc. Such detection methods are particularly useful to achieve simultaneous scanning of multiple attached template nucleic acids.

A number of approaches can be used to detect incorporation of fluorescently-labeled nucleotides into a single nucleic acid molecule. Optical setups include near-field scanning microscopy, far-field confocal microscopy, wide-field epi-illumination, light scattering, dark field microscopy, photoconversion, single and/or multiphoton excitation, spectral wavelength discrimination, fluorophor identification, evanescent wave illumination, and total internal reflection fluorescence (TIRF) microscopy. In general, certain methods involve detection of laser-activated fluorescence using a microscope equipped with a camera. Suitable photon detection systems include, but are not limited to, photodiodes and intensified CCD cameras. For example, an intensified charge couple device (ICCD) camera can be used. The use of an ICCD camera to image individual fluorescent dye molecules in a fluid near a surface provides numerous advantages. For example, with an ICCD optical setup, it is possible to acquire a sequence of images (movies) of fluorophores.

Some embodiments of the present invention use TIRF microscopy for imaging. TIRF microscopy uses totally internally reflected excitation light and is well known in the art. See, e.g., the World Wide Web at nikon-instruments.jp/eng/page/products/tirf.aspx. In certain embodiments, detection is carried out using evanescent wave illumination and total internal reflection fluorescence microscopy. An evanescent light field can be set up at the surface, for example, to image fluorescently-labeled nucleic acid molecules. When a laser beam is totally reflected at the interface between a liquid and a solid substrate (e.g., a glass), the excitation light beam penetrates only a short distance into the liquid. The optical field does not end abruptly at the reflective interface, but its intensity falls off exponentially with distance. This surface electromagnetic field, called the "evanescent wave", can selectively excite fluorescent molecules in the liquid near the interface. The thin evanescent optical field at the interface provides low background and facilitates the detection of single molecules with high signal-to-noise ratio at visible wavelengths.

The evanescent field also can image fluorescently-labeled nucleotides upon their incorporation into the attached template/primer complex in the presence of a polymerase. Total internal reflectance fluorescence microscopy is then used to visualize the attached template/primer duplex and/or the incorporated nucleotides with single molecule resolution.

Some embodiments of the invention use non-optical detection methods such as, for example, detection using nanopores (e.g., protein or solid state) through which molecules are individually passed so as to allow identification of the molecules by noting characteristics or changes in various properties or effects such as capacitance or blockage current flow (see, for example, Stoddart et al, Proc. Nat. Acad. Sci., 106:7702, 2009; Purnell and Schmidt, ACS Nano, 3:2533, 2009; Branton et al, Nature Biotechnology, 26:1146, 2008; Polonsky et al, U.S. Application 2008/0187915; Mitchell & Howorka, Angew. Chem. Int. Ed. 47:5565, 2008; Borsenberger et al, J. Am. Chem. Soc., 131, 7530, 2009) ; or other suitable non-optical detection methods.

### Analysis

Alignment and/or compilation of sequence results obtained from the image stacks produced as generally described above utilizes look-up tables that take into account possible sequences changes (due, e.g., to errors, mutations, etc.). Essentially, sequencing results obtained as described herein are compared to a look-up type table that contains all possible reference sequences plus 1 or 2 base errors.

### Determining presence of female fetal nucleic acid in the maternal sample

Methods of the invention provide for further quantitative or qualitative analysis of the sequence data to detect presence of fetal nucleic acid, regardless of the ability to detect the Y chromosome, particularly for detecting a female fetus in a maternal sample. Generally, the obtained sequences are aligned to a reference genome (e.g., a maternal genome, a paternal genome, or an external standard representing the numerical range considered to be indicative of a normal). Once aligned, the obtained sequences are quantified to determine the number of sequence reads that align to each chromosome. The chromosome counts are assessed and deviation from a 2X normal ratio provides evidence of female fetal nucleic acid in the maternal sample, and also provides evidence of fetal nucleic acid that represents chromosomal aneuploidy.

Numerous different types of quantitative analysis may be performed to detect presence of fetal nucleic acid from a female fetus in the maternal sample. Such additional analysis may include copy number analysis, sparse allele calling, targeted resequencing, differential DNA modification (e.g., methylation, or modified bases), and breakpoint analysis. In certain embodiments, analyzing the sequence data for presence of a portion of the Y chromosome is not required, and methods of the invention may involve performing a quantitative analysis as described herein in order to detect presence of fetal nucleic acid in the maternal sample.

One method to detect presence of fetal nucleic acid from a female fetus in a maternal sample involves performing a copy number analysis of the generated sequence data. This method involves determining the copy number change in genomic segments relative to reference sequence information. The reference sequence information may be a maternal sample known not to contain fetal nucleic acid (such as a buccal sample) or may be an external standard representing the numerical range considered to be indicative of a normal, intact karyotype. In this method, an enumerative amount (number of copies) of a target nucleic acid (i.e., chromosomal DNA or portion thereof) in a sample is compared to an enumerative amount of a reference nucleic acid. The reference number is determined by a standard (i.e., expected) amount of the nucleic acid in a normal karyotype or by comparison to a number of a nucleic acid from a non-target chromosome in the same sample, the non-target chromosome being known or suspected to be present in an appropriate number (i.e., diploid for the autosomes) in the sample. Further description of copy number analysis is shown in Lapidus et al. (U.S. patent numbers 5,928,870 and 6,100,029) and Shuber et al. (U.S. patent number 6,214,558).

The normal human genome will contain only integral copy numbers (e.g., 0, 1, 2, 3, etc.), whereas the presence of fetal nucleic acid in the sample will introduce copy numbers at fractional values (e.g., 2.1). If the analysis of the sequence data provides a collection of copy number measurements that deviate from the expected integral values with statistical significance (i.e., greater than values that would be obtained due to sampling variance, reference inaccuracies, or sequencing errors), then the maternal sample contains fetal nucleic acid. For greater sensitivity, a sample of maternal and/or paternal nucleic acid may be used to provide additional reference sequence information. The sequence information from the maternal and/or paternal sample allows for identification of copy number values in the maternal sample suspected to contain fetal nucleic acid that do not match the maternal control sample and/or match the paternal sample, thus indicating the presence of fetal nucleic acid.

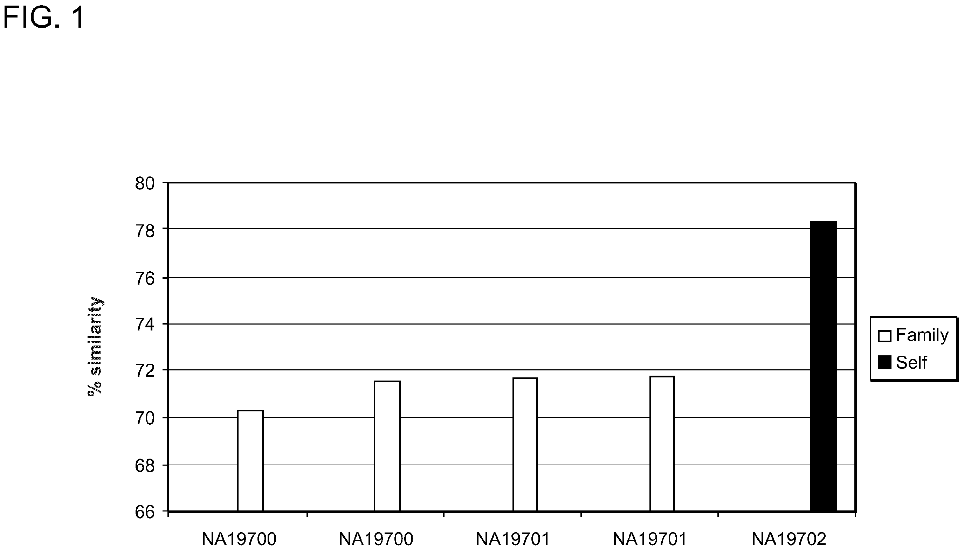

Another method to detect presence of fetal nucleic acid from a female fetus in a maternal sample involves performing sparse allele calling. Sparse allele calling is a method that analyzes single alleles at polymorphic sites in low coverage DNA sequencing (e.g., less than 1x coverage) to compare variations in nucleic acids in a sample. The genome of an individual generally has about three billion base pairs of sequence. For a typical individual, about two million positions are heterozygous and about one million positions are homozygous non-reference single nucleotide polymorphisms (SNPs). If two measurements of the same allele position are compared within an individual they will agree almost 100% of the time in the case of a homozygous position or almost 50% of the time in the case of a heterozygous position (sequencing errors may slightly diminish these numbers). If two measurements of the same allele position are compared within different individuals they will agree less often, depending on the frequency of the different alleles in the population, and the relation between the individuals. The degree of agreement across a wide set of allele positions in two samples is therefore indicative of the relation between the individuals from which the samples were taken, where the closer the relation the higher the agreement (a sample of a sibling or child, for example, will be more similar to an individual's sample than a stranger, but less similar than a second sample from the same individual). Figure 1 shows histograms of the difference between two samples from one individual ("self') and samples of that individual and two family members ("family") representing the comparison of a set of known single nucleotide variants between the different samples.

The method described above can be utilized for detection of fetal DNA in a maternal sample by comparison of this sample to a sample including only maternal DNA (e.g., a buccal sample) an/or a paternal DNA. This method involves obtaining sequence information at low coverage (e.g., less than 1x coverage) to determine whether fetal nucleic acid is present in the sample. The method utilizes the fact that variants occur throughout the genome with millions annotated in publicly available databases. Low coverage allows for analysis of a different set of SNPs in each comparison. The difference between the genome of a fetus and his/her mother is expected to be statistically significant if one looks for differences across a substantial number of the variants found in the maternal genome. In addition, the similarity between the genome of the fetus and the parental DNA is expected to be statistically significant, in comparison to a pure maternal sample, since the fetus inherits half of its DNA for its father.

The invention involves comparing low coverage genomic DNA sequence (e.g., less than 1x coverage) from both the maternal sample suspected to contain fetal DNA and a pure maternal sample, at either known (from existing databases) or suspected (from the data) positions of sequence variation, and determining whether that difference is higher than would be expected if two samples were both purely maternal (i.e. did not contain fetal DNA). A sample of the paternal DNA is not required, but could be used for additional sensitivity, where the paternal sample would be compared to both pure maternal sample and sample with suspected fetal DNA. A statistically significant higher similarity between the suspected sample and paternal sample would be indicative of the presence of fetal DNA.

Another method to detect presence of fetal nucleic acid from a female fetus in a maternal sample involves performing targeted resequencing. Resequencing is shown for example in Harris (U.S. patent application numbers 2008/0233575, 2009/0075252, and 2009/197257). Briefly, a specific segment of the target is selected (for example by PCR, microarray, or MIPS) prior to sequencing. A primer designed to hybridize to this particular segment, is introduced and a primer/template duplex is formed. The primer/template duplex is exposed to a polymerase, and at least one detectably labeled nucleotide under conditions sufficient for template dependent nucleotide addition to the primer. The incorporation of the labeled nucleotide is determined, as well the identity of the nucleotide that is complementary to a nucleotide on the template at a position that is opposite the incorporated nucleotide.

After the polymerization reaction, the primer may be removed from the duplex. The primer may be removed by any suitable means, for example by raising the temperature of the surface or substrate such that the duplex is melted, or by changing the buffer conditions to destabilize the duplex, or combination thereof. Methods for melting template/primer duplexes are well known in the art and are described, for example, in chapter 10 of Molecular Cloning, a Laboratory Manual, 3.sup.rd Edition, J. Sambrook, and D. W. Russell, Cold Spring Harbor Press (2001), the teachings of which are incorporated herein by reference.

After removing the primer, the template may be exposed to a second primer capable of hybridizing to the template. In one embodiment, the second primer is capable of hybridizing to the same region of the template as the first primer (also referred to herein as a first region), to form a template/primer duplex. The polymerization reaction is then repeated, thereby resequencing at least a portion of the template.

Targeted resequencing of highly variable genomic regions allows deeper coverage of those regions (e.g., 1Mb at 100x coverage). Normal human genomes will contain single nucleotide variants at about 100% or about 50% frequencies, whereas presence of fetal nucleic acid will introduce additional possible frequencies (e.g., 10%, 60%, 90%, etc.). If the analysis of the resequence data provides a collection of sequence variant frequencies that deviate from 100% or 50% with statistical significance (i.e., greater than values that would be obtained due to sampling variance, reference inaccuracies, or sequencing errors), then the maternal sample contains fetal nucleic acid.

Another method to detect presence of fetal nucleic acid from a female fetus in a maternal sample involves performing an analysis that looks at breakpoints. A sequence breakpoint refers to a type of mutation found in nucleic acids in which entire sections of DNA are inverted, shuffled or relocated to create new sequence junctions that did not exist in the original sequence. Sequence breakpoints can be identified in the maternal sample suspected to contain fetal nucleic acid and compared with either maternal and/or paternal control samples. The appearance of a statistically significant number of identified breakpoints that are not detected in the maternal control sample and/or detected in the paternal sample, indicates the presence of fetal nucleic acid.

### Detecting fetal abnormalities

Ability to detect fetal nucleic acid in a maternal sample allows for development of a noninvasive diagnostic assay to assess whether a fetus has an abnormality. Thus, another aspect of the invention provides noninvasive methods that analyze fetal nucleic acid in a maternal sample to determine whether a fetus has an abnormality. Methods of the invention involve obtaining a sample including both maternal and fetal nucleic acids, performing a sequencing reaction on the sample to obtain sequence information nucleic acids in the sample, comparing the obtained sequence information to sequence information from a reference genome, thereby determining whether the fetus has an abnormality. In certain embodiments, the reference genome may be the maternal genome, the paternal genome, or a combination thereof. In other embodiments, the reference genome may be an external standard representing the numerical range considered to be indicative of a normal, intact karyotype, such as the currently existing HG18 human reference genome.

A variety of genetic abnormalities may be detected according to the present methods, including aneuplody (i.e., occurrence of one or more extra or missing chromosomes) or known alterations in one or more genes, such as, CFTR, Factor VIII (F8 gene), beta globin, hemachromatosis, G6PD, neurofibromatosis, GAPDH, beta amyloid, and pyruvate kinase. The sequences and common mutations of those genes are known. Other genetic abnormalities may be detected, such as those involving a sequence which is deleted in a human chromosome, is moved in a translocation or inversion, or is duplicated in a chromosome duplication, in which the sequence is characterized in a known genetic disorder in the fetal genetic material not present in the maternal genetic material. For example chromosome trisomies may include partial, mosaic, ring, 18, 14, 13, 8, 6, 4 etc. A listing of known abnormalities may be found in the OMIM Morbid map, http://www.ncbi.nlm.nih.gov/Omim/getmorbid.cgi, the contents of which are incorporated by reference herein in their entirety.

These genetic abnormalities include mutations that may be heterozygous and homozygous between maternal and fetal nucleic acid, and to aneuploidies. For example, a missing copy of chromosome X (monosomy X) results in Turner's Syndrome, while an additional copy of chromosome 21 results in Down Syndrome. Other diseases such as Edward's Syndrome and Patau Syndrome are caused by an additional copy of chromosome 18, and chromosome 13, respectively. The present method may be used for detection of a translocation, addition, amplification, transversion, inversion, aneuploidy, polyploidy, monosomy, trisomy, trisomy 21, trisomy 13, trisomy 14, trisomy 15, trisomy 16, trisomy 18, trisomy 22, triploidy, tetraploidy, and sex chromosome abnormalities including but not limited to XO, XXY, XYY, and XXX.

Examples of diseases where the target sequence may exist in one copy in the maternal DNA (heterozygous) but cause disease in a fetus (homozygous), include sickle cell anemia, cystic fibrosis, hemophilia, and Tay Sachs disease. Accordingly, using the methods described here, one may distinguish genomes with one mutation from genomes with two mutations.

Sickle-cell anemia is an autosomal recessive disease. Nine-percent of US African Americans are heterozygous, while 0.2% are homozygous recessive. The recessive allele causes a single amino acid substitution in the beta chains of hemoglobin.

Tay-Sachs Disease is an autosomal recessive resulting in degeneration of the nervous system. Symptoms manifest after birth. Children homozygous recessive for this allele rarely survive past five years of age. Sufferers lack the ability to make the enzyme N-acetyl-hexosaminidase, which breaks down the GM2 ganglioside lipid.

Another example is phenylketonuria (PKU), a recessively inherited disorder whose sufferers lack the ability to synthesize an enzyme to convert the amino acid phenylalanine into tyrosine Individuals homozygous recessive for this allele have a buildup of phenylalanine and abnormal breakdown products in the urine and blood.

Hemophilia is a group of diseases in which blood does not clot normally. Factors in blood are involved in clotting. Hemophiliacs lacking the normal Factor VIII are said to have Hemophilia A, and those who lack Factor IX have hemophilia B. These genes are carried on the X chromosome, so sequencing methods of the invention may be used to detect whether or not a fetus inherited the mother's defective X chromosome, or the father's normal allele.

A listing of gene mutations for which the present methods may be adapted is found at http://www.gdb.org/gdb, The GDB Human Genome Database, The Official World-Wide Database for the Annotation of the Human Genome Hosted by RTI International, North Carolina USA.

Chromosome specific primers are shown in Hahn et al. (U.S. patent application number 2005/0164241). Primers for the genes may be prepared on the basis of nucleotide sequences obtained from databases such as GenBank, EMBL and the like. For example, there are more than 1,000 chromosome 21 specific primers listed at the NIH UniSTS web site, which can be located at http://www.ncbi.nlm.nih.gov/entrez/query.fcgi?db=unists.

An important aspect of a diagnostic assay is ability of the assay to distinguish between false negatives (no detection of fetal nucleic acid) and true negatives (detection of nucleic acid from a healthy fetus). Methods of the invention provide this capability by detecting presence of at least a portion of a Y chromosome in the sample, and also conducting an additional analysis if the Y chromosome is not detected in the sample. In certain embodiments, methods of the invention distinguish between false negatives and true negatives regardless of the ability to detect the Y chromosome.

If the Y chromosome is detected in the maternal sample, methods of the invention assure that the assay is functioning properly, because the Y chromosome is associated only with males and will be present in a maternal sample only if male fetal nucleic acid is present in the sample. Thus, if no abnormality is detected in the maternal sample, and at least a portion of the Y chromosome is detected in the sample, one can confidently conclude that the assay has detected a fetus (because presence of Y chromosome in a maternal sample is indicative of male fetal nucleic acid), and that the fetus does not include the genetic abnormality for which the assay was conducted.

Methods of the invention also provide for further quantitative or qualitative analysis to detect presence of fetal nucleic acid regardless of the ability to detect the Y chromosome. This step is particularly useful in embodiments in which the sample includes normal nucleic acids from a female fetus. Such additional quantitative analysis may include copy number analysis, sparse allele calling, targeted resequencing, and breakpoint analysis, each of which is discussed above. Thus, if no abnormality is detected in the maternal sample, and quantitative analysis of the sample reveals presence of fetal nucleic acid, one can confidently conclude that the assay has detected a fetus, and that the fetus does not include the genetic abnormality for which the assay was conducted.

### Tagging

In certain aspects, method of the invention determine whether a fetus has an abnormality by obtaining a maternal sample including both maternal and fetal nucleic acids; attaching unique tags to nucleic acids in the sample, in which each tag is associated with a different chromosome; performing a sequencing reaction on the tagged nucleic acids to obtain tagged sequences; and determining whether the fetus has an abnormality by quantifying the tagged sequences.

Attaching tags to target sequences is shown in Kahvejian et al. (U.S. patent application number 2008/0081330), and Steinman et al. (International patent application number PCT/US09/64001). The tag sequence generally includes certain features that make the sequence useful in sequencing reactions. For example the tags are designed to have minimal or no homopolymer regions, i.e., 2 or more of the same base in a row such as AA or CCC, within the unique portion of the tag. The tags are also designed so that they are at least one edit distance away from the base addition order when performing base-by-base sequencing, ensuring that the first and last base do not match the expected bases of the sequence.

The tags may also include blockers, e.g. chain terminating nucleotides, to block base addition to the 3'-end of the template nucleic acid molecules. The tags are also designed to have minimal similarity to the base addition order, e.g., if performing a base-by-base sequencing method generally bases are added in the following order one at a time: C, T, A, and G. The tags may also include at least one non-natural nucleotide, such as a peptide nucleic acid or a locked nucleic acid, to enhance certain properties of the oligonucleotide.

The unique sequence portion of the tag (unique portion) may be of different lengths. Methods of designing sets of unique tags is shown for example in Brenner et al. (U.S. patent number 6,235,475). In certain embodiments, the unique portion of the tag ranges from about 5 nucleotides to about 15 nucleotides. In a particular embodiment, the unique portion of the tag ranges from about 4 nucleotides to about 7 nucleotides. Since the unique portion of the tag is sequenced along with the template nucleic acid molecule, the oligonucleotide length should be of minimal length so as to permit the longest read from the template nucleic acid attached. Generally, the unique portion of the tag is spaced from the template nucleic acid molecule by at least one base (minimizes homopolymeric combinations).

The tag also includes a portion that is used as a primer binding site. The primer binding site may be used to hybridize the now bar coded template nucleic acid molecule to a sequencing primer, which may optionally be anchored to a substrate. The primer binding sequence may be a unique sequence including at least 2 bases but likely contains a unique order of all 4 bases and is generally 20-50 bases in length. In a particular embodiment, the primer binding sequence is a homopolymer of a single base, e.g. polyA, generally 20 - 70 bases in length.

The tag also may include a blocker, e.g., a chain terminating nucleotide, on the 3'-end. The blocker prevents unintended sequence information from being obtained using the 3'-end of the primer binding site inadvertently as a second sequencing primer, particularly when using homopolymeric primer sequences. The blocker may be any moiety that prevents a polymerase from adding bases during incubation with a dNTPs. An exemplary blocker is a nucleotide terminator that lacks a 3'-OH, i.e., a dideoxynucleotide (ddNTP). Common nucleotide terminators are 2',3'-dideoxynucleotides, 3'-aminonucleotides, 3'-deoxynucleotides, 3'-azidonucleotides, acyclonucleotides, etc. The blocker may have attached a detectable label, e.g. a fluorophore. The label may be attached via a labile linkage, e.g., a disulfide, so that following hybridization of the bar coded template nucleic acid to the surface, the locations of the template nucleic acids may be identified by imaging. Generally, the detectable label is removed before commencing with sequencing. Depending upon the linkage, the cleaved product may or may not require further chemical modification to prevent undesirable side reactions, for example following cleavage of a disulfide by TCEP the produced reactive thiol is blocked with iodoacetamide.

Methods of the invention involve attaching the tag to the template nucleic acid molecules. Template nucleic acids are able to be fragmented or sheared to desired length, e.g. generally from 100 to 500 bases or longer, using a variety of mechanical, chemical and/or enzymatic methods. DNA may be randomly sheared via sonication, e.g. Covaris method, brief exposure to a DNase, or using a mixture of one or more restriction enzymes, or a transposase or nicking enzyme. RNA may be fragmented by brief exposure to an RNase, heat plus magnesium, or by shearing. The RNA may be converted to cDNA before or after fragmentation.

In certain embodiments, the tag is attached to the template nucleic acid molecule with an enzyme. The enzyme may be a ligase or a polymerase. The ligase may be any enzyme capable of ligating an oligonucleotide (RNA or DNA) to the template nucleic acid molecule. Suitable ligases include T4 DNA ligase and T4 RNA ligase (such ligases are available commercially, from New England Biolabs. In a particular embodiment. Methods for using ligases are well known in the art. The polymerase may be any enzyme capable of adding nucleotides to the 3' terminus of template nucleic acid molecules. The polymerase may be, for example, yeast poly(A) polymerase, commercially available from USB. The polymerase is used according to the manufacturer's instructions.

The ligation may be blunt ended or via use of complementary over hanging ends. In certain embodiments, following fragmentation, the ends of the fragments may be repaired, trimmed (e.g. using an exonuclease), or filled (e.g., using a polymerase and dNTPs), to form blunt ends. Upon generating blunt ends, the ends may be treated with a polymerase and dATP to form a template independent addition to the 3'-end of the fragments, thus producing a single A overhanging. This single A is used to guide ligation of fragments with a single T overhanging from the 5'-end in a method referred to as T-A cloning.

Alternatively, because the possible combination of overhangs left by the restriction enzymes are known after a restriction digestion, the ends may be left as is, i.e., ragged ends. In certain embodiments double stranded oligonucleotides with complementary over hanging ends are used. In a particular example, the A:T single base over hang method is used (see Figures 1-2).

In a particular embodiment, the substrate has anchored a reverse complement to the primer binding sequence of the oligonucleotide, for example 5'-TC CAC TTA TCC TTG CAT CCA TCC TCT GCC CTG or a polyT(50). When homopolymeric sequences are used for the primer, it may be advantageous to perform a procedure known in the art as a "fill and lock". When polyA (20-70) on the sample and polyT (50) on the surface hybridize there is a high likelihood that there will not be perfect alignment, so the hybrid is filled in by incubating the sample with polymerase and TTP. Following the fill step, the sample is washed and the polymerase is incubated with one or two dNTPs complementary to the base(s) used in the lock sequence. The fill and lock can also be performed in a single step process in which polymerase, TTP and one or two reversible terminators (complements of the lock bases) are mixed together and incubated. The reversible terminators stop addition during this stage and can be made functional again (reversal of inhibitory mechanism) by treatments specific to the analogs used. Some reversible terminators have functional blocks on the 3'-OH which need to be removed while others, for example Helicos BioSciences Virtual Terminators have inhibitors attached to the base via a disulfide which can be removed by treatment with TCEP.

Once, tagged, the nucleic acids from the maternal sample are sequenced as described herein. The tags allow for template nucleic acids from different chromosomes to be differentiated from each other throughout the sequencing process. Because, the tags are each associated with a different chromosome, the tagged sequences can be quantified. The sequence reads are assessed for any deviation from a 2X normal ratio, which deviation indicates a fetal abnormality.

In one alternative, cell-free maternal nucleic acid is barcoded prior to sequencing by ligating barcode sequences to the 3' end of the maternal DNA fragments. A preferred barcode is 5 to 8 nucleotides, which are used as unique identifiers of maternal cell-free DNA. Those sequences may also include a 50nt polynucleotide (e.g.., Poly-A) tail. Doing this allows subsequent hybridization of the nucleic acid directly to the flow cell surface followed by sequencing. Among other things, this method allows the combination of different maternal DNA samples into a single flow cell channel for sequencing, thus allowing the reactions to be multiplexed.

### Detecting unique sequences

In certain aspects, method of the invention are used to detect fetal nucleic acid by obtaining a maternal sample suspected to include fetal nucleic acid, detecting at least two unique sequences in the sample, and determining whether fetal nucleic acid is present in the maternal sample based on the ratio of the detected sequences to each other. The unique sequences are sequences known to occur only once in the relevant genome (e.g., human) and can be known unique k-mers or can be determined by sequencing. Advantageously, these methods of the invention do not require comparison to a reference sequence. In a maternal sample, two or more unique k-mers would be expected to occur in identical frequency, leading to a ration of 1.0. A statistically-significant variance from the expected ration is indicative of the presence of fetal nucleic acid in the sample.

In certain embodiments, one or more unique k-mer sequences are predetermined based on available knowledge of the unique k-mers in the human genome. For example, it is possible to estimate the number of unique k-mers in any genome based upon the consensus sequence. Knowledge of the actual occurrence of unique sequences of any given number of bases is readily available to those of ordinary skill in the relevant art.

In one embodiment, a count is made of the number of times that any two or more unique sequences are detected in the maternal sample. For example, sequence A (e.g., a unique 20-mer) may be detected 80 times and sequence B (e.g., a unique 30-mer) may be detected 100 times. If the sequence is uniformly detected across the human genome, or at least for the portion(s) that include sequences A and B, then fetal nucleic acid having sequence B is present in the maternal sample at a level above the maternal background indicated at least in part by the ratio of (100 - 80) to 80. To the extent that sequence is not uniformly detected, various known methods of statistical analysis may be employed to determine whether the measured difference between the frequency of sequence A and sequence B is statistically significant.

Also, either sequence A, B, or both may be selected to have content (e.g., GC rich) such that uniform detection is more likely based on factors known to those of ordinary skill in the art. A large number of unique sequences may be selected in order to make the statistical comparison more robust. Moreover, the sequences may be selected based on their location in a genomic region of particular interest. For example, sequences may be selected because of their presence in a chromosome associated with aneuploidy. Thus, in certain embodiments, if sequence A (detected 80 times) had been selected based on its location not in a chromosome associated with aneuploidy, and sequence B (detected 100 times) had been selected based on its location within a chromosome associated with aneuploidy, a diagnosis of fetal aneuploidy could be made.

In other embodiments, the unique sequences include one or more known SNPs at known locations. In addition to counting the number of times that sequence A is detected in the maternal sample, the number of times may also be counted that sequence A has one variant at a known SNP location (for example, a "G") and the number of times that sequence A has the other variant at that SNP location (e.g., a "T"). As long as both the mother and the fetus are not homozygous for the same base at that location, fetal signal may be detected by any deviation of either G or T from the levels statistically likely (to any desired level of certainty) assuming any other combination of zygosity. For the case in which both mother and fetus are homozygous at the SNP location, a comparison with another one or more predetermined unique sequences (such as sequence B) may be made as previously described..

In yet another approach, detected sequences need not be unique and need not be predetermined. Moreover, there is no need to know anything about the human (or other) genome. Rather, a signature of the mother may be distinguished from a signature of the fetus (if present) based on a pattern of n-mers (or n-mers and k-mers, etc.). For example, in any pattern of n-mers, there will be SNPs, such that the mother has one base (e.g., "G") and the fetus, if present, has another base (e.g., "T") in at least one of the two alleles. If all n-mers (in a sufficiently large sample in view of any error rate) have a "G," then it can be said that there is no fetal nucleic acid. If some statistically significant number of n-mers have a "T" at the SNP location, then fetal nucleic acid has been detected and the amount, relative to the mother's nucleic acid, can be determined. This is true even though there may be two or more places where the n-mer occurs in either or both of the mother's or fetus' genomes (*i.e.,* the sequences are not unique), because, given a large enough number of reads, there will be a statistically significant difference in detected SNPs based on the presence or lack of fetal signal. That is, there will be a statistically significant difference in the frequency of alleles that are detected between what would be expected from only one contributing organism rather than two (or more).

### EXAMPLES

### Example 1: Determining presence of fetal nucleic acid in a sample

Samples of nucleic acid from lymphocytes were obtained from normal healthy adult males and females. Nucleic acids were extracted by protocols known in the art. The sample set included 2 HapMap trios (6 samples) run in 8 HELISCOPE Sequencer channels (Single molecule sequencing instrument, Helicos BioSciences Corporation) on 3 different machines (2 technical replicates). Genomic DNA from one of the samples was sequenced in each channel (8-13M uniquely aligned reads).

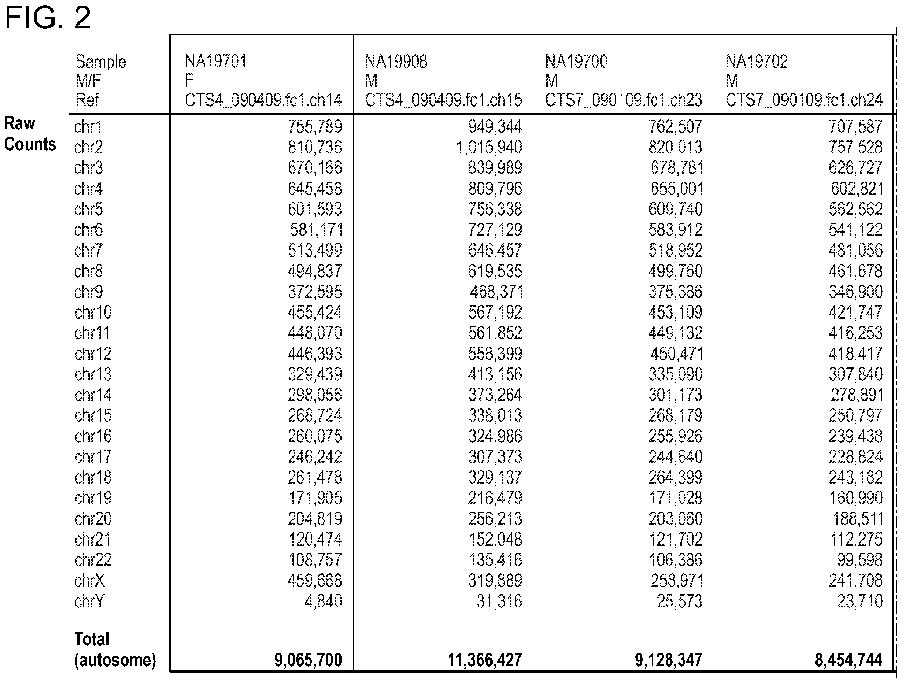

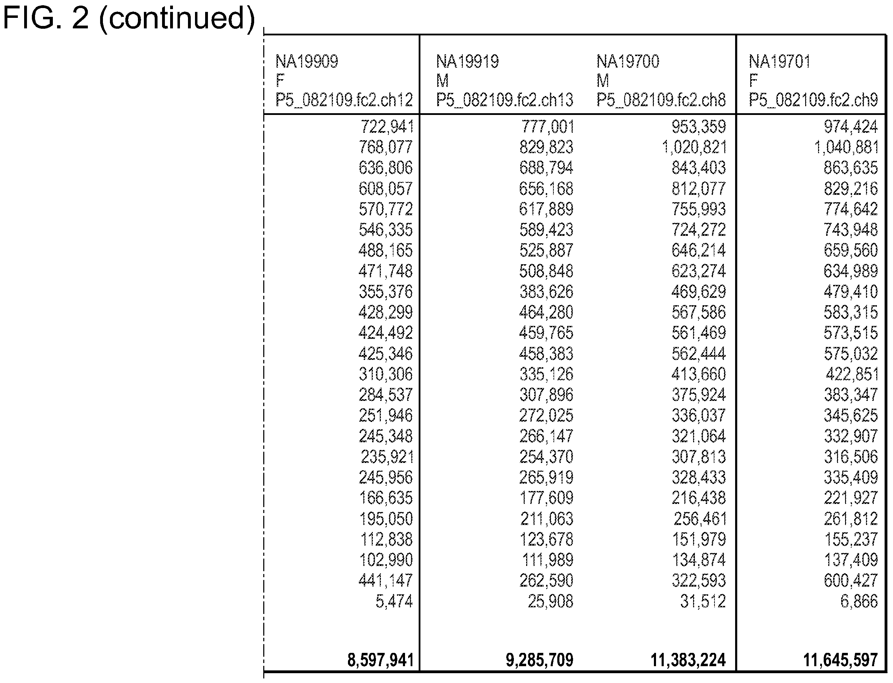

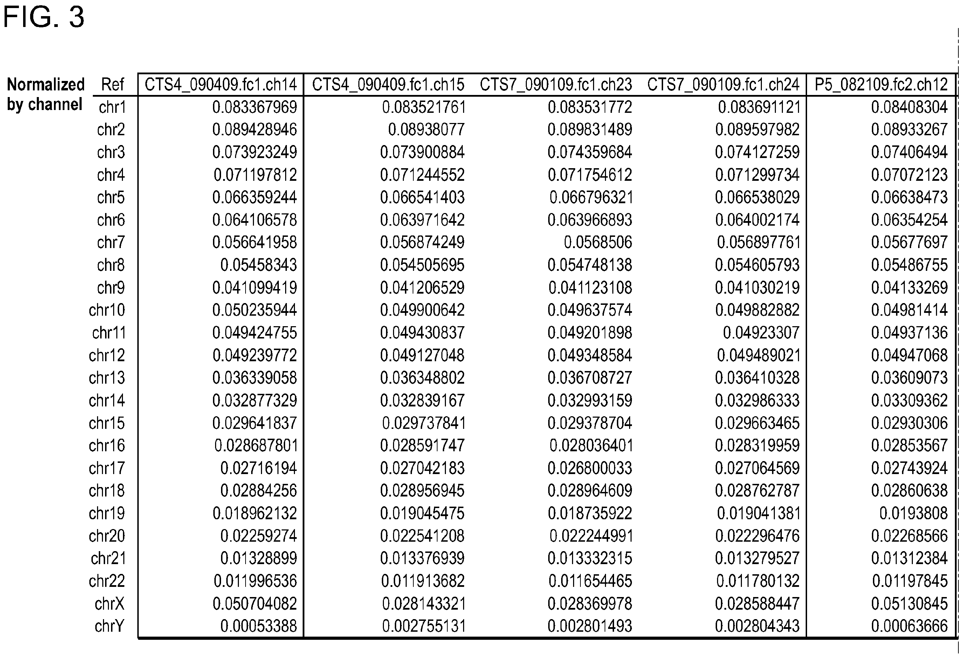

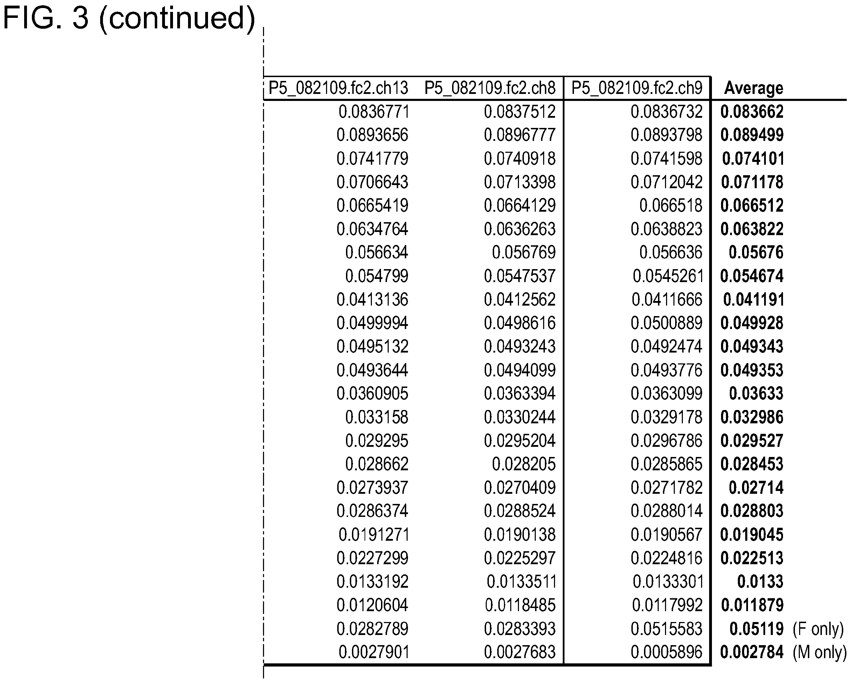

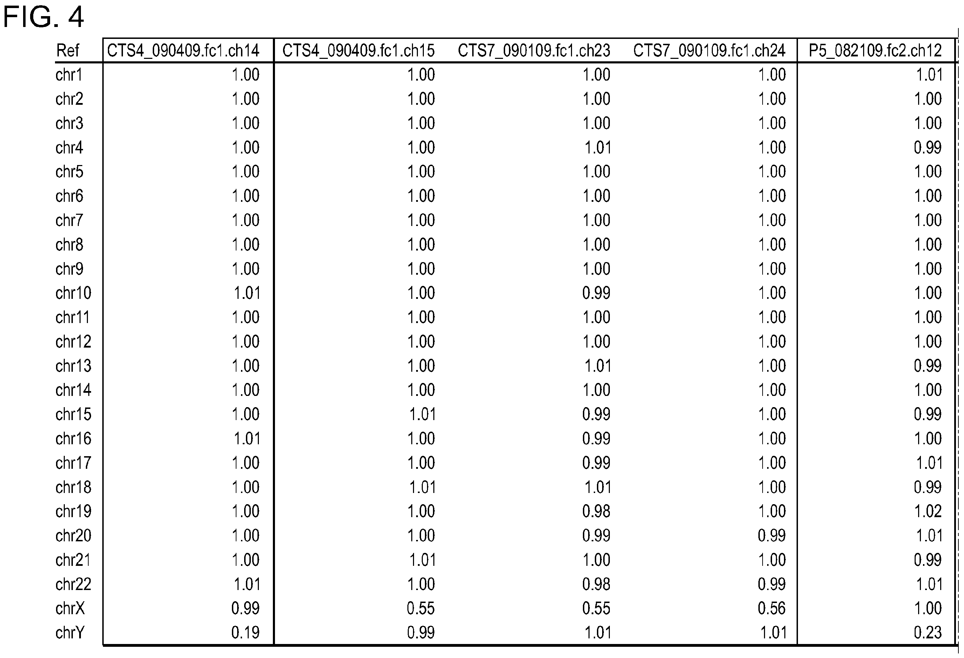

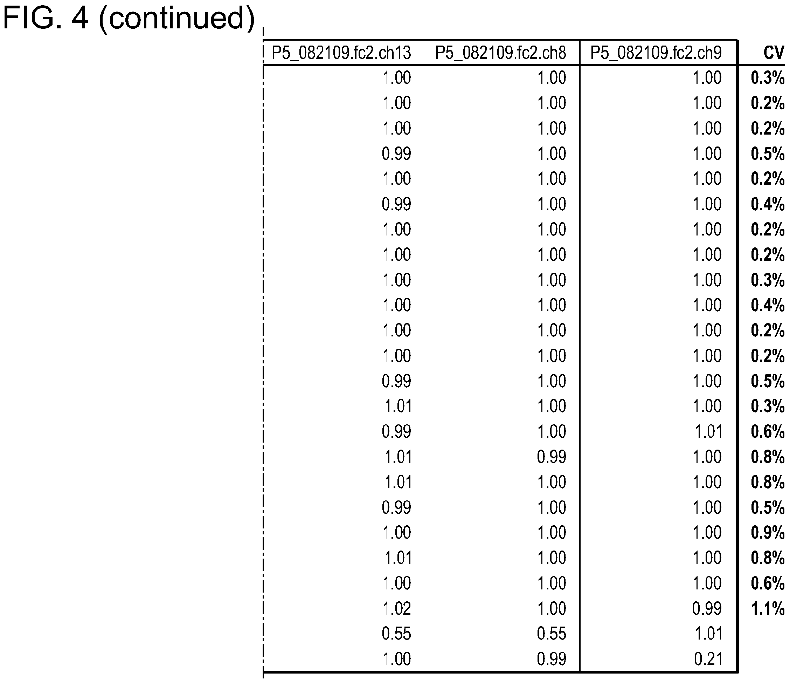

The dataset includes 8 compressed files, one for each HELISCOPE channel. The sequence reads were mapped to a reference human genome, and reads with non-unique alignments were discarded (Figure 2). Counts were first normalized per sample, based on the total counts to the autosomal chromosomes (Figure 3). Counts were then normalized per chromsome, based on the average fraction of reads aligned to each chromosome across all samples (chrX - females only, chrY - males only; Figure 4).

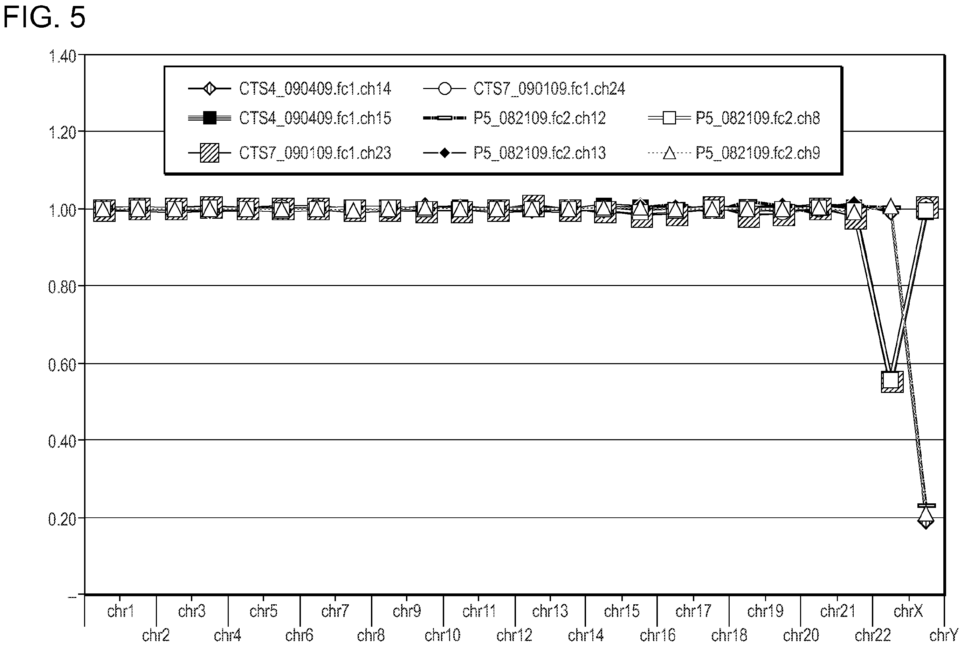

Data show quantitative chromosomal analysis (Figure 5). These data show the genomic sequencing of selected HapMap samples, both male and female, followed by accurate quantitation of the chromosomal counts. Data herein show the distinct ability to identify expected ratios of chromosome X and chromosome Y. The data derived from genomic DNA obtained from individuals, demonstrate the evenness of genomic coverage expected from a normal diploid genome, and demonstrate that no fetal nucleic acid is found in these samples. The deviation in the normalized counts per chromosome is 0.5% CV on average. It is lower (0.2-0.3%) for the larger chromosomes and higher (0.8-1.1%) for the smaller chromosomes. Female and Male samples are clearly distinguishable.

### Example 2: Detecting fetal nucleic acid in a maternal sample and detecting trisomy

Maternal cell free plasma nucleic acid was obtained using methods well known in the art, such as a Qiagen nucleic acid purification kit. The nucleic acid was then subjected to the following protocol. Briefly, the protocol consists of a one hour 3' polyA tailing step, followed by a one hour 3' dideoxy-blocking step. The protocol was performed with 500pg of nucleic acid.

### Required reagents

| | |
|---|---|
| Terminal Transferase kit | NEB M0315 |
| dATP | Roche 11277049001 |
| Biotin-ddATP | Perkin Elmer NEL548001 |
| Carrier Oligonucleotide | 50-mer oligonucleotide |
| Bovine Serum Albumin | NEB B9001S |
| Nuclease-free water | |
| Quant-iT™ PicoGreen dsDNA Reagent | Invitrogen P11495 |

### Required equipment

Pre-chilled Aluminum Block milled for 0.2 mL tubes
Thermocycler
P-2, P20, P200 Pipette
Ice bucket
Nanodrop 3300 or a standard plate reader for the PicoGreen assay

### Methods

Prior to conducting the tailing reaction on the DNA, RNA contamination was removed using RNase digestion and cleanup with a Qiagen Reaction Cleanup Kit (catalog 28204). DNA should was accurately quantitated prior to use. The Quant-iT™ PicoGreen dsDNA Reagent Kit (Invitrogen, catalog#P11495) with a Nanodrop 3300 Fluorospectrometer was used. Molecular biology-grade nuclease-free glycogen or linear acrylamide was used as carrier during DNA clean-up/precipitation steps.

The following mix was prepared: NEB Terminal Transferase 10X buffer (2µl); 2.5mM CoCl₂ (2µl); and maternal cell free plasma nucleic acid and Nuclease-free water (10.8µl). The total volume was 14.8µl. The mix was heated at 95°C for 5 minutes in the thermocycler to denature the DNA. After heating, the mix was cooled on the pre-chilled aluminum block that was kept in an ice and water slurry (about 0°C) to obtain single-stranded DNA. The sample was chilled as quick as possible to prevent re-annealing of the denatured, single-stranded DNA.

On ice, the following mix was added to the denatured DNA from above: 1µl of Terminal Transferase (dilute 1:4 to 5U/µl using 1x buffer); 4µl of 50µM dATP; and 0.2µl of BSA. The volume of this mix was 5.2µl, bringing the total volume of the reaction to 20µl. The tubes containing the mixture were placed in the thermocycler and the following program was run: 37°C for 1 hour; 70°C for 10 minutes; and temperature was brought back down to 4°C. A poly(A) tail will now have been added to the DNA.

The 20µl poly-adenylation reaction was denature by heating the mixture to 95°C for 5 minutes in the thermocycler followed by rapid cooling in the pre-chilled aluminum block kept in an ice and water slurry (about 0°C). The sample was chilled as quick as possible to prevent re-annealing of the denatured, single-stranded DNA.

The following blocking mixture was added to the denatured poly-adenylated mixture from above: 1µl of Terminal Transferase 10X buffer; 1µl of CoCl₂ (2.5mM); 1µl of Terminal Transferase (dilute 1:4 to 5U/µl using 1x buffer); 0.5µl of 200µM Biotin-ddATP; and 6.5µl of nuclease-free water. The volume of this mix was 10µl, bringing the total volume of the reaction to 30µl.

The tubes containing the mixture were placed in the thermocycler and the following program was run: 37°C for 1 hour; 70°C for 20 minutes; and temperature was brought back down to 4°C. It was observed that that a 3' end block was now added to the poly-adenylated DNA.

2 picomoles of control oligonucleotide was added to the heat inactivated 30µl terminal transferase reaction above. The control oligonucleotide was added to the sample to minimize DNA loss during sample loading steps. The control oligonucleotide does not contain a poly(A) tail, and therefore will not hybridize to the flow cell surface. The sample is now ready to be hybridized to the flow cells for the sequencing reaction. No additional clean-up step is required.

The samples were loaded into HELISCOPE Sequencer channels (Single molecule sequencing instrument, Helicos BioSciences Corporation) according to the manufacturer's instructions. DNA from the sample was sequenced in the channels according to the manufacturer's instructions. The sequence reads were mapped to a reference human genome, and reads with non-unique alignments were discarded. Counts were first normalized per sample, based on the total counts to the autosomal chromosomes. Counts were then normalized per chromsome, based on the average fraction of reads aligned to each chromosome across all samples (chrX - females only, chrY - males only). Chromosome counts for chromosomes 1, 18, and 21 across the samples were compared to deviations from the expected values based on control samples.

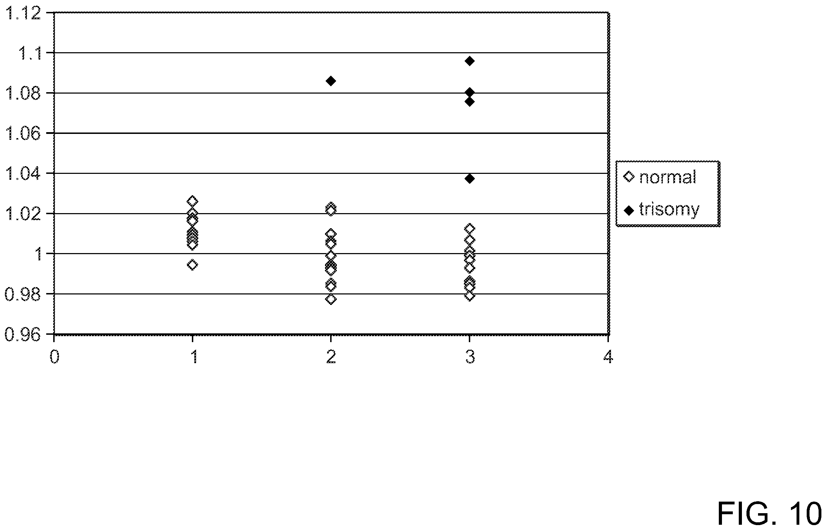

Figure 10 shows results of analysis of the sequence information. In this Figure, chromosome 1 was used as a control. Data herein show that fetal DNA was detected (Figure 10). Data herein further show that trisomy of chromosome 18 and chromosome 21 was also detected (Figure 10).

### Example 3: Correcting for GC bias

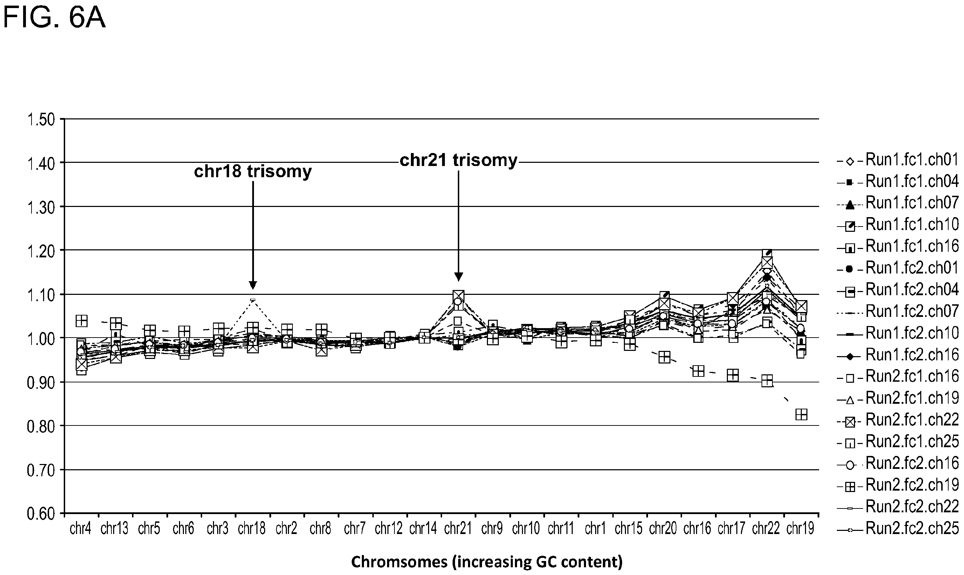

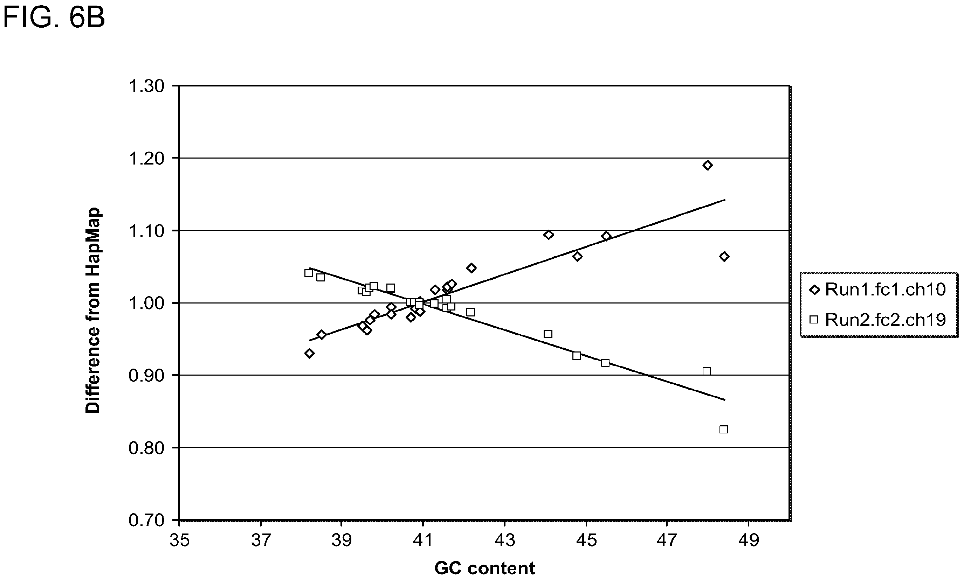

When performing chromosomal counting analysis base on sequencing information (i.e., quantifying the amount of each chromosome, or chromosome segment, based on relative representation) a relative number of read counts of each chromosome (or chromosome segment) are compared to a standard measured across one or more normal samples. Certain steps in the sample preparation or sequencing process may result in a GC bias, where the relative representation of each chromosome is influenced not only by the relative quantity (copy number) of that chromosome, but also by its GC content. A difference in GC bias between the measured sample and the control (normal) sample will result in skewing of the chromosomal counts such that chromosomes with extreme GC content may appear to have more or fewer than their real copy number. Figure 6 is a graph showing a sample in which chromosomal counts are skewed by GC bias. The chromosomes are ordered by increasing GC content. These data show that variability of measurement is higher for chromosomes with extreme GC content.

Methods of the invention allow for determining an amount of GC bias in obtained sequence information, and also allow for correction of the GC bias in the sequence information. In certain embodiments, methods of the invention involve sequencing a sample to obtain nucleic acid sequence information; determining an amount of GC bias in the sequence information; correcting the sequence information to account for the GC bias; and analyzing the corrected information.

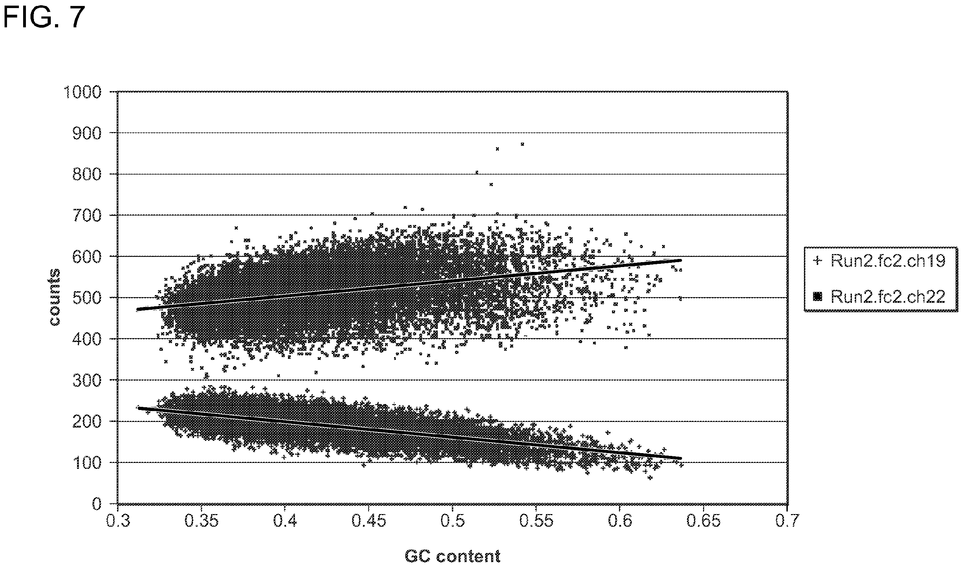

Determining the amount of GC bias in a sample may be accomplished in numerous ways. In certain embodiments, the amount of GC bias may be quantified by partitioning the genome into bins, and measuring the correlation between the number of counts in each bin and its GC content. Figure 7 is a graph showing counts in each bin plotted as a function of GC content of the bin. In this embodiment, the genome is partitioned into 1000kbp bins. Although this number is exemplary and any size may be used. A significant negative or positive correlation indicates the existence of GC bias (see Figure 7). In Figure 7, the upper sample shows positive correlation with GC content, and the lower sample shows negative correlation with GC content.

Methods of the invention reduce or eliminate the effects of GC bias in sequence information.

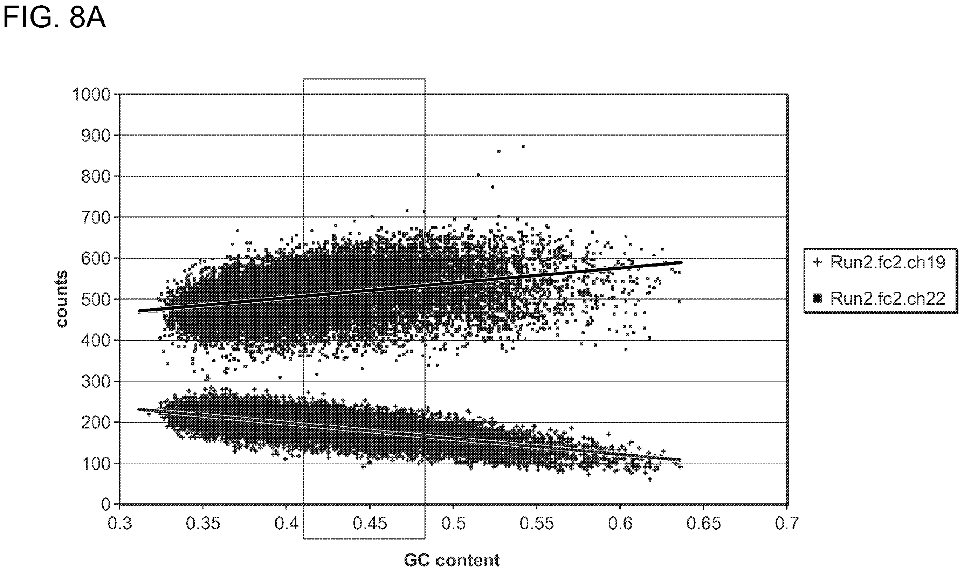

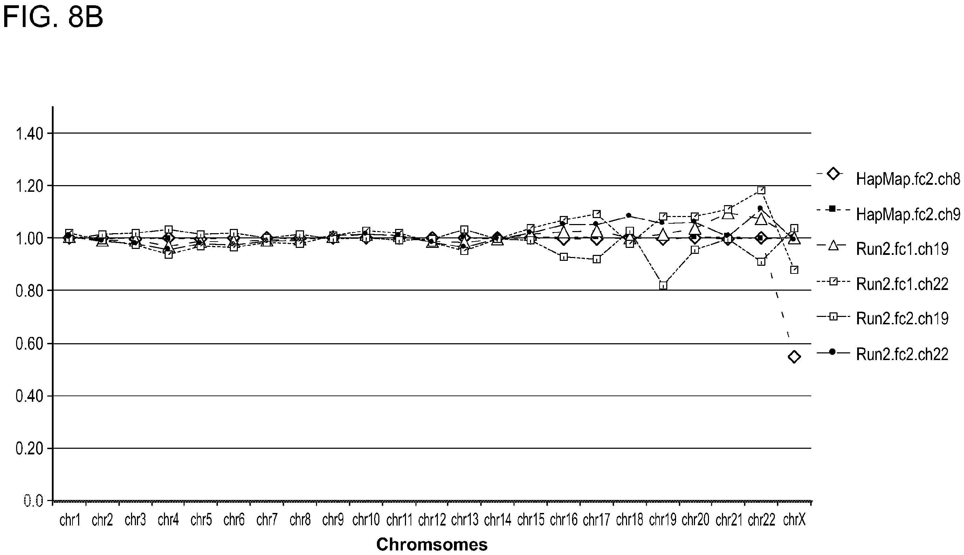

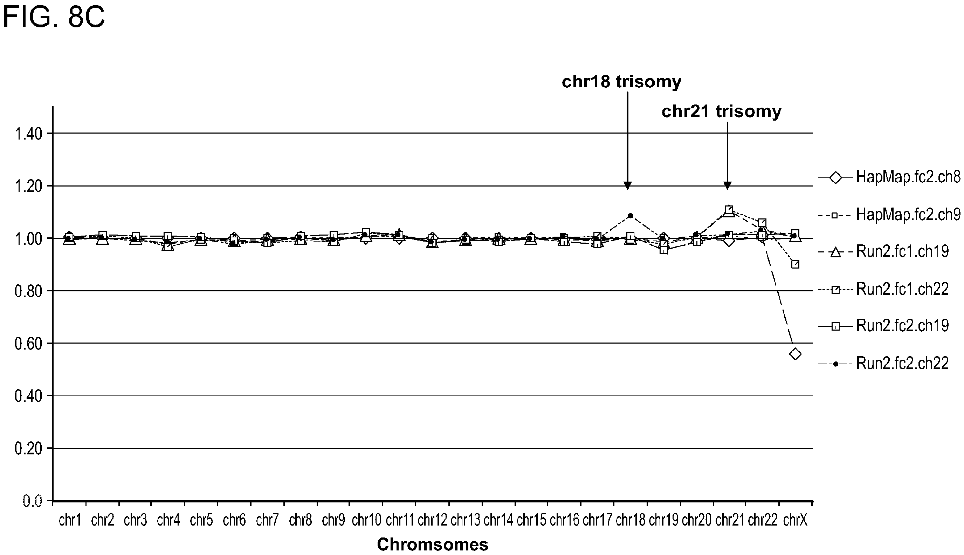

A subset of genomic bins is selected within a given range such that the average GC content per chromosome is equalized (or less skewed). Chromosomal counting is then performed on the selected subset. Figure 8 provides an example of this protocol. In Figure 8, analysis was limited to only genomic bins with a given GC content of 0.42 to 0.48, approximately 25% of the genome (Figure 8 panel A)

Figure 8 panels B and C show the difference in obtained sequence information after there is a correction for GC bias in the sequence information. Figure 8 panel B shows the sequence information prior to correction for GC bias. Figure 8 panel C shows the sequence information after correction for GC bias. These data show that the GC bias was skewing the chromosomal counts such that chromosomes with extreme GC content appeared to have more or fewer than their real copy number. After correction for GC bias in the sequence information, the data show a more accurate chromosomal count, and allowed for the detection of trisomy at chromosome 18 and 21, which was not possible from analysis of the sequence information prior to correction for GC bias.

Disclosed, but outside the scope of the claims, is a method in which the correlation between GC content and chromosome counts is modeled across a set of genomic bins using a mathematical function (e.g. a first or second order polynomial). An exemplary mathematical function is a regression model (i.e., fitting the sequence information to a mathematical function, such as lower order functions (linear and/or quadratic polynomials)). The effect of GC bias is corrected for by subtracting the GC-dependent component, reflected by the model, from the count of each bin. Chromosomal counting is then performed based on the corrected counts. An advantage is that it retains the number of counts of the original dataset, which is important for the sensitivity of the method.

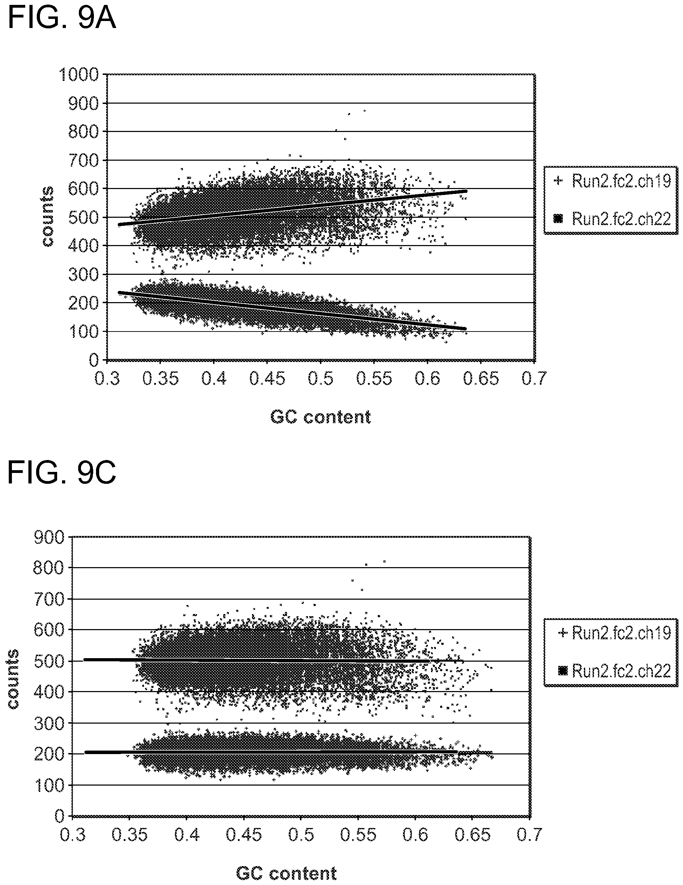

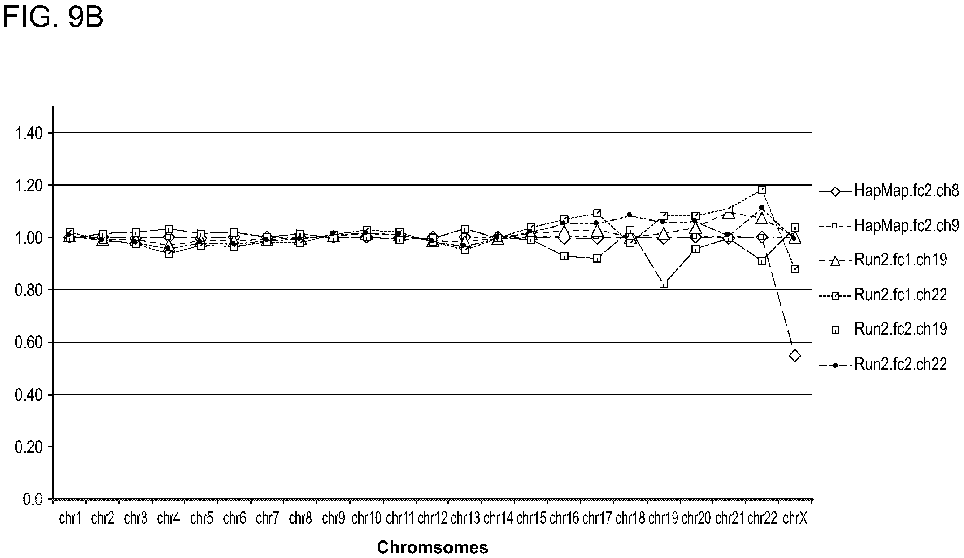

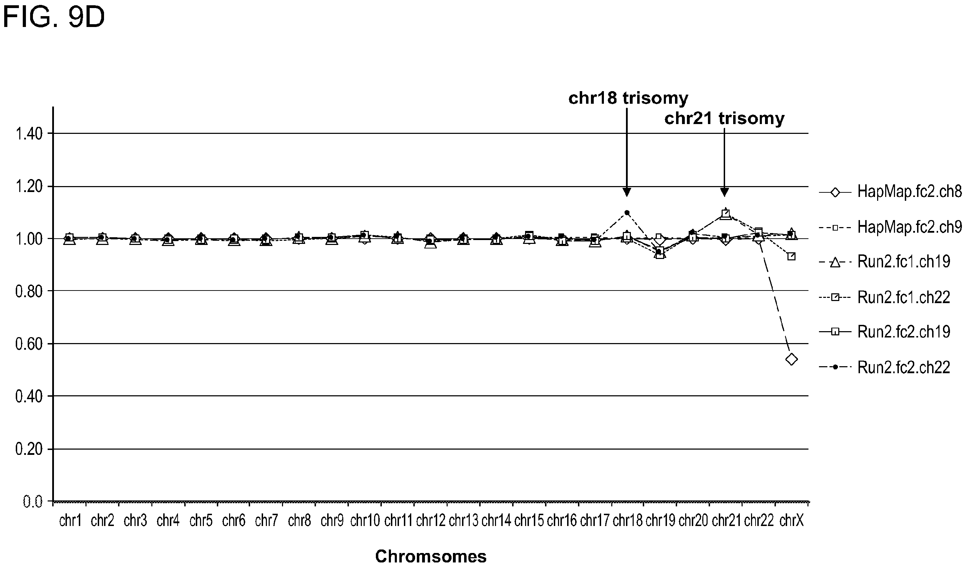

Figure 9 provides an example of this protocol. In Figure 9, the sequence information was corrected by subtracting a linear model of GC dependence from each genomic bin. Figure 9 panels A and B show sequence information prior to correction for GC bias. Figure 9 panels C and D show sequence information after correction for GC bias. These data show that the GC bias was skewing the chromosomal counts such that chromosomes with extreme GC content appeared to have more or fewer than their real copy number. After correction for GC bias in the sequence information, the data show a more accurate chromosomal count, and allowed for the detection of trisomy at chromosome 18 and 21, which was not possible from analysis of the sequence information prior to correction for GC bias.

Disclosed, but outside the scope of the claims, is a method in which GC bias is corrected for as follows. An average coverage per bin over a number of control samples is obtained, and the observed coverage in the sample is divided by the mean of the control population (this could be a weighted mean to take into account different levels of overall coverage in the control samples). Each corrected bin value would then be a ratio of observed to expected, which will be more consistent across bins of different %GC.

## Claims

1. A method for analyzing nucleic acids in a sample, the method comprising:
sequencing a sample to obtain nucleic acid sequence information;
determining an amount of GC bias in the sequence information, wherein the determining comprises:
partitioning the genome into bins; and
measuring a correlation between a number of counts in each bin and its GC content, wherein a statistically significant negative or positive correlation indicates existence of GC bias;
correcting the sequence information to account for the GC bias by selecting a subset of bins within a given range of GC content such that average GC content per chromosome is equalized; and
analyzing the corrected sequence information by performing chromosomal counting using the counts of the selected subset of bins.

2. The method according to claim 1, comprising
comparing corrected sequence information to a reference sequence;
identifying fetal nucleic acid in the sample;
determining whether the fetus has an abnormality based on the comparison to the reference sequence.

3. The method according to claim 1 or 2, wherein the bins in the selected subset of bins have a range of GC content of about 0.42 to 0.48.

4. The method according to claim 1 or 2, wherein the selected subset of bins represents about 25 % of a reference genome.

5. The method according to any one of claims 1 to 4, wherein the size of a bin is about 1000 kilobase pairs (kb).

6. The method according to any one of claims 1 to 5, wherein the sample is a tissue or body fluid.

7. The method according to claim 6, wherein the sample is suspected to contain fetal nucleic acid.

8. The method according to claim 7, wherein the body fluid is maternal blood, blood plasma, or serum.

9. The method according to any one of claims 1 to 8, wherein sequencing is single molecule sequencing and/or sequencing by nanopore detection.

10. The method according to claim 2, wherein the reference sequence is selected from a maternal reference sequence, a fetal reference sequence, or a consensus human genomic sequence.

11. The method according to claim 10, wherein said maternal reference sequence is selected from a sequence obtained from a buccal sample, a saliva sample, a urine sample, a breast nipple aspirate sample, a sputum sample, a tear sample, and an amniotic fluid sample.

12. The method according to claim 2, wherein the fetal nucleic acid is cell free circulating fetal nucleic acid.

13. The method according to claim 2, wherein prior to the sequencing step, the method further comprises enriching for fetal nucleic acid in the sample.

14. The method according to claim 2, wherein the identifying step comprises a technique selected from sparse allele calling, targeted gene sequencing, identification of Y chromosomal material, enumeration, copy number analysis, and inversion analysis.

## Patentansprüche

1. Verfahren zum Analysieren von Nukleinsäuren in einer Probe, wobei das Verfahren umfasst:
Sequenzieren einer Probe, um Nukleinsäuresequenzinformation zu erhalten;
Bestimmen einer Menge von GC-Verschiebung in der Sequenzinformation, wobei das Bestimmen umfasst:
Unterteilen des Genoms in distinkte Bereiche; und
Messen einer Korrelation zwischen einer Anzahl von Zählern in einem jeden distinkten Bereich und seinem GC-Gehalt, wobei eine statistisch signifikante negative oder positive Korrelation das Vorhandensein von GC-Verschiebung anzeigt;
Korrigieren der Sequenzinformation, um die GC-Verschiebung zu erfassen, durch Auswählen einer Untergruppe von distinkten Bereichen innerhalb eines gegebenen Bereiches an GC-Gehalt, so dass der durchschnittliche GC-Gehalt pro Chromosom angeglichen wird; und
Analysieren der korrigierten Sequenzinformation durch Durchführen von Chromosomenzählen unter Verwendung der Zähler der ausgewählten Untergruppe von distinkten Bereichen.

2. Verfahren nach Anspruch 1, umfassend
Vergleichen von korrigierter Sequenzinformationen mit einer Referenzsequenz; Identifizieren von fötaler Nukleinsäure in der Probe;
Bestimmen auf der Grundlage des Vergleichs mit der Referenzsequenz, ob der Fötus eine Anomalität aufweist.

3. Verfahren nach Anspruch 1 oder 2, wobei die distinkten Bereiche in der ausgewählten Untergruppe von distinkten Bereichen einen GC-Gehaltbereich von etwa 0,42 bis 0,48 aufweisen.

4. Verfahren nach Anspruch 1 oder 2, wobei die ausgewählte Untergruppe von distinkten Bereichen etwa 25% eines Referenzgenoms darstellt.

5. Verfahren nach einem der Ansprüche 1 bis 4, wobei die Größe eines distinkten Bereiches etwa 1000 Kilobasenpaar (kb) beträgt.

6. Verfahren nach einem der Ansprüche 1 bis 5, wobei die Probe ein Gewebe oder eine Körperflüssigkeit ist.

7. Verfahren nach Anspruch 6, wobei angenommen wird, dass die Probe fötale Nukleinsäure enthält.

8. Verfahren nach Anspruch 7, wobei die Körperflüssigkeit mütterliches Blut, Blutplasma oder Serum ist.

9. Verfahren nach einem der Ansprüche 1 bis 8, wobei Sequenzieren Einzelmolekülsequenzierung und/oder Sequenzierung durch Nanoporennachweis ist.

10. Verfahren nach Anspruch 2, wobei die Referenzsequenz ausgewählt ist aus einer mütterlichen Referenzsequenz, einer fötalen Referenzsequenz oder einer humanen genomischen Konsenssequenz.

11. Verfahren nach Anspruch 10, wobei die mütterliche Referenzsequenz ausgewählt ist aus einer Sequenz, die von einer Wangenprobe, einer Speichelprobe, einer Urinprobe, einer Probe von Brustwarzenaspirat, einer Sputumprobe, einer Tränenprobe und einer Fruchtwasserprobe erhalten ist.

12. Verfahren nach Anspruch 2, wobei die fötale Nukleinsäure zellfreie zirkulierende fötale Nukleinsäure ist.

13. Verfahren nach Anspruch 2, wobei das Verfahren vor dem Sequenzierungsschritt weiter Anreichern fötaler Nukleinsäure in der Probe umfasst.

14. Verfahren nach Anspruch 2, wobei der Identifizierungsschritt eine Technik umfasst, die ausgewählt ist aus Bestimmung seltener Allele, Targetgensequenzierung, Identifizierung von Y-chromosomalem Material, Enumeration, Kopienanzahlanalyse und Inversionsanalyse.

## Revendications

1. Procédé d'analyse d'acides nucléiques dans un échantillon, le procédé comprenant :
le séquençage d'un échantillon pour obtenir des informations de séquence d'acide nucléique ;
la détermination d'une quantité de biais de GC dans les informations de séquence, dans lequel la détermination comprend :
la séparation du génome en fenêtres ; et
la mesure d'une corrélation entre un nombre de comptages dans chaque fenêtre et sa teneur en GC, dans lequel une corrélation négative ou positive statistiquement significative indique l'existence d'un biais de GC ;
la correction des informations de séquence pour prendre en considération le biais de GC par sélection d'un sous-ensemble de fenêtres dans une plage donnée de teneur en GC de telle sorte que la teneur moyenne en GC par chromosome est égalisée ; et
l'analyse des informations de séquence corrigées en réalisant un comptage chromosomique en utilisant les comptages du sous-ensemble sélectionné de fenêtres.

2. Procédé selon la revendication 1, comprenant
la comparaison des informations de séquence corrigées à une séquence de référence ;
l'identification d'un acide nucléique foetal dans l'échantillon ;
la détermination du fait que le foetus présente ou non une anomalie sur la base de la comparaison à la séquence de référence.

3. Procédé selon la revendication 1 ou 2, dans lequel les fenêtres dans le sous-ensemble de fenêtres sélectionné ont une plage de teneur en GC d'environ 0,42 à 0,48.

4. Procédé selon la revendication 1 ou 2, dans lequel le sous-ensemble sélectionné de fenêtres représente environ 25 % d'un génome de référence.

5. Procédé selon l'une quelconque des revendications 1 à 4, dans lequel la taille d'une fenêtre est d'environ 1 000 paires de kilobases (kb).

6. Procédé selon l'une quelconque des revendications 1 à 5, dans lequel l'échantillon est un fluide tissulaire ou corporel.

7. Procédé selon la revendication 6, dans lequel l'échantillon est soupçonné de contenir un acide nucléique foetal.

8. Procédé selon la revendication 7, dans lequel le fluide corporel est du sang maternel, du plasma sanguin, ou du sérum.

9. Procédé selon l'une quelconque des revendications 1 à 8, dans lequel le séquençage est le séquençage d'une molécule unique et/ou le séquençage par détection par nanopores.

10. Procédé selon la revendication 2, dans lequel la séquence de référence est sélectionnée parmi une séquence de référence maternelle, une séquence de référence foetale, ou une séquence génomique humaine consensus.

11. Procédé selon la revendication 10, dans lequel ladite séquence de référence maternelle est sélectionnée parmi une séquence obtenue d'un échantillon buccal, d'un échantillon de salive, d'un échantillon d'urine, d'un échantillon d'aspirat de mamelon, d'un échantillon de crachat, d'un échantillon de larmes, et d'un échantillon de liquide amniotique.

12. Procédé selon la revendication 2, dans lequel l'acide nucléique foetal est un acide nucléique foetal circulant acellulaire.

13. Procédé selon la revendication 2, dans lequel avant l'étape de séquençage, le procédé comprend en outre l'enrichissement en acide nucléique foetal dans l'échantillon.

14. Procédé selon la revendication 2, dans lequel l'étape d'identification comprend une technique sélectionnée parmi la dénomination d'allèles rares, le séquençage de gènes ciblés, l'identification de matériau du chromosome Y, l'énumération, l'analyse du nombre de copies, et l'analyse d'inversion.
